(19) Europäisches Patentamt / European Patent Office / Office européen des brevets

(11) **EP 3 039 418 B1**

(12) **EUROPÄISCHE PATENTSCHRIFT**

(45) Veröffentlichungstag und Bekanntmachung des Hinweises auf die Patenterteilung:
**21.06.2023 Patentblatt 2023/25**

(21) Anmeldenummer: **14758123.5**

(22) Anmeldetag: **28.08.2014**

(51) Internationale Patentklassifikation (IPC):
**A61B 5/0515** (2021.01)   **G01N 27/74** (2006.01)
**G01R 33/12** (2006.01)   *G01N 15/06* (2006.01)

(52) Gemeinsame Patentklassifikation (CPC):
**G01R 33/1276; A61B 5/0515; G01N 27/745;
G01R 33/1223;** G01N 15/0656

(86) Internationale Anmeldenummer:
**PCT/EP2014/068303**

(87) Internationale Veröffentlichungsnummer:
**WO 2015/028569 (05.03.2015 Gazette 2015/09)**

(54) **VERFAHREN UND VORRICHTUNG ZUR ANALYSE EINES MAGNETISCHE PARTIKEL UMFASSENDEN PROBENVOLUMENS**

METHOD AND APPARATUS FOR ANALYSING A SAMPLE VOLUME COMPRISING MAGNETIC PARTICLES

PROCÉDÉ ET DISPOSITIF D'ANALYSE D'UN VOLUME D'ÉCHANTILLON COMPORTANT DES PARTICULES MAGNÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2013 DE 102013109467**

(43) Veröffentlichungstag der Anmeldung:
**06.07.2016 Patentblatt 2016/27**

(73) Patentinhaber: **Fraunhofer-Gesellschaft zur Förderung
der angewandten Forschung e.V.
80686 München (DE)**

(72) Erfinder: **FIDLER, Florian
97072 Würzburg (DE)**

(74) Vertreter: **Aurigium Leischner & Luthe
Patentanwälte Partnerschaft mbB
Robert-Koch-Straße 2
82152 Planegg (DE)**

(56) Entgegenhaltungen:
EP-A2- 1 262 766       WO-A1-2007/122293
WO-A1-2008/145813    DE-A1- 19 946 656
US-A- 5 001 424         US-A- 6 110 660
US-A1- 2012 164 916

• GLEICH AND J WEIZENECKER B: "Tomographic imaging using the nonlinear response of magnetic particles", NATURE, NATURE PUBLISHING GROUP, UNITED KINGDOM, Bd. 435, Nr. 7046, 30. Juni 2005 (2005-06-30), Seiten 1214-1217, XP002368326, ISSN: 0028-0836, DOI: 10.1038/NATURE03808 in der Anmeldung erwähnt

**Beschreibung**

Die vorliegende Erfindung bezieht sich auf ein Verfahren und auf eine Vorrichtung zur Analyse eines magnetische Partikel umfassenden Probenvolumens.

Nanopartikel mit magnetischen Eigenschaften finden in Technik und Medizin ein breites Anwendungsgebiet. Sie können als Marker für bestimmte Eigenschaften wie etwa die Vitalität von Zellen, als Kontrastmittel oder als Tracer zur Darstellung von Fluss oder Gefäßen benutzt werden. Die magnetischen Eigenschaften können indirekt oder direkt gemessen werden.

Die US 2012/0164916 A1, die WO2008145813 A1 und die WO 2007/122293 A1 offenbaren Vorrichtungen zum Sensieren einer Konzentration von magnetischen Partikeln. Dazu wird eine Serienschaltung aus zwei Spulen eingesetzt. An die äußeren Anschlüsse der Serienschaltung wird eine Wechselspannung als Anregungssignal angelegt und ein Knotenpunkt zwischen den zwei Spulen dient als eine Messschnittstelle.

Ein Beispiel für ein indirektes Messverfahren ist zum Beispiel die Magnetresonanz-Tomografie (MRT), bei der Eisen- oder Gadoliniumhaltige Substanzen als Kontrastmittel eingesetzt werden. Hier erfolgt der Nachweis in der Regel über die veränderten Relaxationseigenschaften der umgebenden Protonen. Dies ermöglicht zum einen die Eigenschaften der Kontrastmittel zu bestimmen aber auch eine Lokalisation vorzunehmen. Es gibt eine Vielzahl solcher Partikel und Kontrastmittel mit magnetischen Eigenschaften, darunter auch eine Reihe funktionalisierter Partikel. Die sind mit speziellen Oberflächenbeschichtungen und/oder Bindungsgliedern versehen, um z. B. an spezielle Zellen zu binden. Finden solche Bindungen statt, so sind zum einen die Partikel an den Targets lokalisiert, können aber auch durch zum Beispiel Clusterbildung ihre Eigenschaften ändern.

Für den direkten Nachweis von magnetischen Partikeln haben Gleich und Weizenecker 2005 in Nature Vol 435/30 p.1214ff ein Verfahren zum Magnetic Particle Imaging (MPI) vorgestellt, das Partikel mit einer nichtlinearen Magnetisierungskurve lokalisieren kann.

Dieses Verfahren basiert darauf, dass die Magnetisierung der Partikel in einem äußeren Wechsel-Magnetfeld nur für kleine Feldstärken nicht-Linearitäten aufweist, für große Feldstärken aber in eine Sättigung gelangt, die dann ein lineares Verhalten zeigt. Über ein Zusatzfeld mit einem entsprechend großen Magnetfeld, das so ausgeformt ist, das es nur in einem kleinen Bereich ein sehr kleines Feld hat bzw. feldfrei ist, der Feld-Freie-Punkt (FFP), zeigen nur die Partikel in diesem Feld-Freien-Punkt ein nicht-lineares Magnetisierungsverhalten. Der Nachweis erfolgt über die Messung der Magnetisierung der Partikel. Finden sich in der Fourier-Zerlegung (Spektrum) höhere Harmonische der Frequenz des Wechselfeldes, so kann daraus auf das Vorhandensein von Partikeln im Feld-Freien-Punkt geschlossen werden und ein Bild durch Abrastern der Probe mit dem Feld-Freien-Punkt berechnet werden. Diese höheren Harmonischen werden zur Auswertung herangezogen.

Es ist die Aufgabe der vorliegenden Erfindung ein verbessertes Verfahren und eine verbesserte Vorrichtung zur Analyse eines magnetische Partikel umfassenden Probenvolumens zu schaffen.

Diese Aufgabe wird durch ein Verfahren und eine Vorrichtung zur Analyse eines magnetische Partikel umfassenden Probenvolumens gemäß den Hauptansprüchen 1 und 7 gelöst.

Der beschriebene Ansatz eignet sich als Nachweisverfahren für magnetische Partikel. Gemäß Ausführungsformen des beschriebenen Ansatzes kann eine Lokalisation und zusätzlich oder alternativ eine Vermessung der Partikel-Eigenschaften durch Messung der magnetischen Permeabilität vorgenommen werden. Vorteilhafterweise ist dabei die Verwendung einer einzigen Spule ausreichend, die sowohl eingesetzt werden kann, um ein auf ein Probenvolumen einwirkendes magnetisches Anregungsfeld zu erzeugen als auch eingesetzt werden kann, um eine Information über eine magnetische Permeabilität des Probenvolumens zu erhalten. Aus der Information über die magnetische Permeabilität des Probenvolumens kann beispielsweise auf das Vorhandensein zumindest eines magnetischen Partikels in dem Probenvolumen geschlossen werden.

Ein Verfahren zur Analyse eines magnetische Partikel umfassenden Probenvolumens, umfasst die Schritte gemäß unabhängigem Anspruch 1.

Magnetfeldlinien des von der Messspule erzeugten magnetischen Felds können durch das Probenvolumen verlaufen. Unter dem Probenvolumen kann ein zu analysierender Bereich oder eine Probe selbst verstanden werden. Beispielsweise kann das Probenvolumen auch eine zu analysierende Fläche umfassen. Gemäß unterschiedlicher Ausführungsformen kann das Probenvolumen im Inneren der Messspule, direkt benachbart zu der Messspule oder beabstandet zu der Messspule angeordnet sein. Je nach Zustand des Probenvolumens können sich in dem Probenvolumen keine magnetischen Partikel oder zumindest ein magnetischer Partikel befinden. Unter einem magnetischen Partikel kann dabei magnetisches Material verstanden werden, das beispielsweise als Probe in das Probenvolumen eingebracht werden kann. Die Messspule kann eine oder eine Mehrzahl von Spulenwicklungen aufweisen. Die Spule kann eine geeignete Form, beispielsweise eine Zylinderform aufweisen. Die Spule kann in einer geeigneten Schaltung, beispielsweise einer Brückenschaltung verschaltet sein. Das Anregungssignal kann direkt an die Messspule oder über ein oder mehrere Elemente an die Messspule angelegt werden. Das Anregungssignal kann ein Wechselsignal sein. Somit kann das magnetische Feld ein Wechselfeld sein. Beispielsweise kann das Anregungssignal eine Wechselspannung oder ein Wechselstrom sein. Das Messsignal kann eine elektrische Spannung repräsentieren. Ein solches Messsignal kann durch eine Spannungsmessung erfasst werden. Beispielsweise kann eine an der Spule abfallende Spannung erfasst werden. Auch kann das Messsignal einen elektrischen Strom repräsentieren. Ein solches Messsignal kann durch eine Strommessung erfasst werden, beispielsweise durch Messen des durch die Spule fließenden Stroms. Die Schritte des Erfassens des Messsignals und des Anlegens des Anregungssignals können zeitgleich ausgeführt werden. Dabei können die Schritte des Erfassens und Anlegens über einen längeren Zeitraum durchgeführt werden. Handelt es sich bei dem Anregungssignal um ein Wechselsignal, so kann das Messsignal beispielsweise erfasst werden, während das Anregungssignal eine oder mehrere Schwingungsphasen durchläuft. Die Induktivität der Spule ist abhängig von der magnetischen Permeabilität des Probenvolumens. Ändert sich die magnetische Permeabilität des Probenvolumens, so ändert sich die Induktivität der Messspule. Somit kann das Messsignal die magnetische Permeabilität oder eine Änderung der magnetischen Permeabilität des Probenvolumens anzeigen. Der Schritt des Analysierens der magnetischen Permeabilität des Probenvolumens kann somit eine Auswertung einer Größe, einer Veränderung oder eines zeitlichen Verlaufs des Messsignals umfassen. Durch die Auswertung des Messsignals kann eine Information über die magnetische Permeabilität des Probenvolumens erhalten werden. Unter der magnetischen Permeabilität des Probenvolumens kann das Verhältnis der magnetischen Flussdichte innerhalb des Probenvolumens zu der magnetischen Feldstärke innerhalb des Probenvolumens verstanden werden. Die Information über die magnetische Permeabilität kann wiederum verwendet werden, um eine magnetische Eigenschaft der sich innerhalb des Probenvolumens befindlichen Materie zu analysieren, beispielsweise zumindest ein magnetisches Partikel in dem Probenvolumen zu detektieren.

Die Schritte des Verfahrens können ausgeführt werden, um die Permeabilität des Probenvolumens zu vermessen. Aus der daraus resultierenden Permeabilitätskurve des Probenvolumens kann die Magnetisierungskurve des Probenvolumens bestimmt werden. Auf diese Weise können sowohl lineare als auch nichtlineare Magnetisierungskurven bestimmt werden.

Das Verfahren kann einen Schritt des Vergleichens des Messsignals mit einem Referenzsignal umfassen. Das Referenzsignal kann wie nachfolgend ausgeführt eine Referenzspannung, beispielsweise aber auch ein Referenzstrom sein. Durch den Vergleich kann die magnetische Permeabilität des Probenvolumens bestimmt werden. Dabei kann das Referenzsignal ein weiteres elektrisches Messsignal repräsentieren, das von der Induktivität der Messspule abhängig ist, während die Messspule ein auf ein Referenzvolumen einwirkendes magnetisches Feld erzeugt. Das Referenzvolumen kann eine Referenzprobe, beispielsweise Luft, enthalten. Somit können das Messsignal und das Referenzsignal zu unterschiedlichen Zeiten unter Verwendung derselben Messspule erfasst werden, einmal während das magnetische Feld auf das Probenvolumen einwirkt (zur Bestimmung des Messsignals) und einmal während das magnetische Feld auf das Referenzvolumen einwirkt (zur Bestimmung des Referenzsignals). Zu beiden Zeiten kann die Messspule von dem gleichen Anregungssignal angeregt werden. Beispielsweise kann das Referenzvolumen mit einem Material einer bekannten magnetischen Eigenschaft, beispielsweise mit Luft gefüllt sein. Somit kann unter dem Probenvolumen beispielsweise ein Volumen verstanden werden, in dem sich eine Probe befindet, und unter dem Referenzvolumen kann dasselbe Volumen verstanden werden, in dem sich jedoch keine Probe befindet. Das Referenzsignal kann einmal erfasst und dann gespeichert werden. Somit kann im Schritt des Vergleichens das Messsignal mit einem gespeicherten Referenzsignal verglichen werden. Der Vergleich kann durch eine geeignete Verknüpfung zwischen dem Messsignal und dem Referenzsignal durchgeführt werden. Beispielsweise kann die magnetische Permeabilität basierend auf einem Unterschied zwischen dem Messsignal und dem Referenzsignal sowie einer dem Referenzsignal zugeordneten magnetischen Permeabilität bestimmt werden. Somit kann die magnetische Permeabilität des Probenvolumens sehr einfach bestimmt werden.

Gemäß einer Ausführungsform, die nicht Teil der Erfindung ist, können ein erster Kontakt der Messspule über ein erstes Element mit einem ersten Anschluss zum Anlegen des elektrischen Anregungssignals, ein zweiter Kontakt der Messspule über ein zweites Element mit einem zweiten Anschluss zum Anlegen eines elektrischen Abgleichsignals und der zweite Kontakt der Messspule mit einer Messschnittstelle verbunden sein. Die Elemente können beispielsweise Durchgangsleitungen, Widerstände, Kondensatoren oder Spulen sein. Ein Element kann auch eine Serienschaltung und/oder Parallelschaltung mehrerer Elemente repräsentieren. Im Schritt des Anlegens kann das Anregungssignal an den ersten Anschluss und das Abgleichsignal an den zweiten Anschluss angelegt werden. Im Schritt des Erfassens kann das Messsignal an der Messschnittstelle erfasst werden. Dabei kann das Abgleichsignal ein Signal repräsentieren, bei dem an der Messschnittstelle eine Referenzspannung anliegt, wenn das von der Messspule erzeugte magnetische Feld auf ein Referenzvolumen einwirkt. Die Referenzspannung kann einen bestimmten Spannungswert, beispielsweise Null Volt, aufweisen. Ein Wert oder Verlauf des Abgleichssignals kann während einer Referenzmessung bestimmt worden sein, bei dem das magnetische Feld der Messspule auf das bereits zuvor genannte Referenzvolumen einwirkt. Dies ermöglicht es, sehr einfache Schaltungen zum Bereitstellen des Anregungssignals und des Abgleichsignals zur Analyse des Probenvolumens zu verwenden.

Gemäß der Erfindung sind ein erster Kontakt der Messspule über ein erstes Element mit einem ersten Anschluss zum Anlegen des elektrischen Anregungssignals, ein zweiter Kontakt der Messspule über ein zweites Element mit einem zweiten Anschluss zum Anlegen eines elektrischen Abgleichsignals und der zweite Kontakt der Messspule mit einer Messschnittstelle verbunden. Im Schritt des Anlegens wird das Anregungssignal an den ersten Anschluss und das Abgleichsignal an den zweiten Anschluss angelegt. Im Schritt des Erfassens wird das Abgleichsignal als das Messsignal erfasst. Dabei ist das Abgleichsignal ausgeformt, um unabhängig davon, ob das von der Messspule erzeugte magnetische Feld auf das Probenvolumen oder ein Referenzvolumen einwirkt, an der Messschnittstelle eine vorbestimmte Referenzspannung einzustellen. Somit wird das Abgleichsignal fortlaufend nachgeregelt, um die bestimmte Referenzspannung einzustellen. Beispielsweise kann die Referenzspannung Null Volt betragen. Vorteilhafterweise ist auf diese Weise keine Referenzmessung erforderlich.

Das Verfahren kann einen Schritt des Anlegens eines weiteren elektrischen Anregungssignals an eine weitere Messspule umfassen, um unter Verwendung der weiteren Messspule ein auf ein weiteres Probenvolumen einwirkendes weiteres magnetisches Feld zu erzeugen. Ferner kann das Verfahren einen Schritt des Erfassens eines von der Induktivität der weiteren Messspule abhängigen weiteren elektrischen Messsignals umfassen. Dabei kann das weitere Messsignal aufgrund der Abhängigkeit der Induktivität der weiteren Messspule von der magnetischen Permeabilität des weiteren Probenvolumens eine Analyse der magnetischen Permeabilität des weiteren Probenvolumens ermöglichen. Die beiden Probenvolumen können identisch sein, sich zumindest teilweise überlagern oder sich nicht überlagern. Überlagern sich die Probenvolumen zumindest teilweise, so kann eine Probe sehr genau analysiert werden. Beispielsweise kann eine räumliche Verteilung magnetischen Materials innerhalb der Probe analysiert werden.

Dabei kann das Verfahren einen Schritt des Bestimmens einer Verteilung der magnetischen Partikel in dem Probenvolumen und dem weiteren Probenvolumen unter Verwendung des Messsignals, des weiteren Messsignals sowie einer Information über eine Position des Probenvolumens, einer Position des weiteren Probenvolumens, einer Information über eine Charakteristik des Anregungssignals und einer Information über eine Charakteristik des weiteren Anregungssignals umfassen. Auf diese Weise kann durch eine geeignete Wahl der Charakteristika der Anregungssignale eine räumliche Verteilung der magnetischen Partikel bestimmt werden.

Zusätzlich oder alternativ kann das Verfahren einen Schritt des Beaufschlagens des Probenvolumens und des weiteren Probenvolumens mit einem zusätzlichen Magnetfeld umfassen. Ferner kann das Verfahren einen Schritt des Bestimmens einer Verteilung der magnetischen Partikel in dem Probenvolumen und dem weiteren Probenvolumen unter Verwendung des Messsignals, des weiteren Messsignals sowie einer Information über eine Charakteristik des zusätzlichen Magnetfelds in dem Probenvolumen und dem weiteren Probenvolumen umfassen. Somit besteht eine weitere Möglichkeit, um die räumliche Verteilung der magnetischen Partikel zu bestimmen.

Gemäß einer Ausführungsform kann das Verfahren einen Schritt des Beaufschlagens des Probenvolumens mit einem inhomogenen zusätzlichen Magnetfeld aufweisen. In dem Schritt des Analysierens kann eine Verteilung der magnetischen Partikel in dem Probenvolumen unter Verwendung des Messsignals sowie einer Information über einen örtlichen Verlauf des zusätzlichen Magnetfelds innerhalb des Probenvolumens bestimmt werden. Die Information über den örtlichen Verlauf kann beispielsweise eine Information über eine Position eines eine geringe Magnetfeldstärke aufweisenden Abschnitts des zusätzlichen Magnetfelds umfassen.

Dabei können die Schritte des Anlegens und des Erfassens sowie des Beaufschlagens mehrmals ausgeführt werden, wobei der örtliche Verlauf des zusätzlichen Magnetfelds in den mehrmaligen Schritten des Beaufschlagens des Probenvolumens verändert werden kann. Im Schritt des Analysierens kann die Verteilung der magnetischen Partikel unter Verwendung der in den mehrmaligen Schritten des Erfassens erfassten Messsignale und einer Information über eine Veränderung des örtlichen Verlaufs des zusätzlichen Magnetfelds während der mehrmaligen Schritte des Beaufschlagens bestimmt werden. Somit können beispielsweise aufeinanderfolgende Schritte des Erfassens jeweils bei einem unterschiedlichen örtlichen Verlauf des zusätzlichen Magnetfelds durchgeführt werden. Unter Kenntnis des örtlichen Verlaufs des zusätzlichen Magnetfelds während der Erfassung der einzelnen Messsignale kann beispielsweise eine räumliche Verteilung der magnetischen Permeabilität oder eine räumliche Verteilung der magnetischen Partikel innerhalb des Probenvolumens bestimmt werden.

Eine Vorrichtung zur Analyse eines magnetische Partikel umfassenden Probenvolumens weist die Merkmale gemäß unabhängigem Anspruch 7 auf.

Eine solche Vorrichtung eignet sich zum Umsetzen der Schritte des genannten Verfahrens zur Analyse eines magnetische Partikel umfassenden Probenvolumens.

Bei einer solchen Vorrichtung kann das Probenvolumen im Inneren der Messspule angeordnet sein. Auf diese Weise kann beispielsweise eine in einem Reagenzglas angeordnete Probe analysiert werden. Alternativ kann das Probenvolumen außerhalb der Messspule angeordnet sein.

Die Vorrichtung kann einen Spulenkern aufweisen, der durch die Messspule geführt ist. Dabei kann ein Ende des Spulenkerns als eine Spitze zum Anlegen an das Probenvolumen ausgeführt sein. Durch die Spitze kann das magnetische Feld der Messspule auf das Probenvolumen konzentriert werden. Zudem kann der Spulenkern verwendet werden, um einen räumlichen Abstand zwischen der Spule und dem Probenvolumen zu überbrücken.

Die Vorrichtung weist eine Auswerteeinrichtung auf. Die Auswerteeinrichtung ist ausgebildet, um die magnetische Permeabilität des Probenvolumens unter Verwendung des Messsignals zu bestimmen. Die magnetische Permeabilität von magnetischen Partikeln ermöglicht sowohl die Analyse der Partikel selbst, als auch deren Umgebung.

Gemäß der Erfindung sind ein erster Kontakt der Messspule über ein erstes Element mit einem ersten Anschluss zum Anlegen des elektrischen Anregungssignals, ein zweiter Kontakt der Messspule über ein zweites Element mit einem zweiten Anschluss zum Anlegen eines elektrischen Abgleichsignals und der zweite Kontakt der Messspule mit der Messschnittstelle verbunden. Auf diese Weise kann die Messspule in eine geeignete Brückenschaltung eingebunden werden.

Dabei kann das erste Element ein Kondensator oder eine elektrische Durchgangsleitung sein. Das zweite Element kann ein Widerstand, eine Spule oder eine elektrische Durchgangsleitung sein. Durch eine geeignete Wahl der Elemente können messtechnische Eigenschaften der Vorrichtung eingestellt werden.

Die Vorrichtung kann eine weitere Messspule aufweisen. Die weitere Messspule kann ausgebildet sein, um ansprechend auf ein Anlegen eines weiteren elektrischen Anregungssignals an die weitere Messspule ein auf ein weiteres Probenvolumen einwirkendes weiteres magnetisches Feld zu erzeugen. Die Vorrichtung kann ferner eine weitere Messschnittstelle zum Erfassen eines von der Induktivität der weiteren Messspule abhängigen weiteren elektrischen Messsignals aufweisen. Dabei kann das weitere Messsignal aufgrund der Abhängigkeit der Induktivität der weiteren Messspule von der magnetischen Permeabilität des weiteren Probenvolumens eine Analyse der magnetischen Permeabilität des weiteren Probenvolumens ermöglichen. Die Probenvolumen können dabei unterschiedlich oder zumindest teilweise identisch sein. Das Probenvolumen und das weitere Probenvolumen können somit zumindest teilweise überlappen. Unter Verwendung der weiteren Messspule kann beispielsweise ein Probenvolumen genauer oder es kann ein größeres Probenvolumen analysiert werden, als unter Verwendung nur einer Messspule. Dabei kann die Vorrichtung zwei, drei oder mehr Messspulen aufweisen.

Bevorzugte Ausführungsbeispiele der vorliegenden Erfindung werden nachfolgend Bezug nehmend auf die beiliegenden Zeichnungen näher erläutert. Es zeigen:

Figuren 1 bis 6    schematische Darstellungen einer Vorrichtung zur Analyse eines Probenvolumens gemäß Ausführungsbeispielen der vorliegenden Erfindung;

Fig. 7    ein Ablaufdiagramm eines Verfahrens zur Analyse eines Probenvolumens gemäß einem Ausführungsbeispiel der vorliegenden Erfindung;

Figuren 8 bis 9    schematische Darstellungen einer Vorrichtung zur Analyse eines Probenvolumens gemäß Ausführungsbeispielen der vorliegenden Erfindung; und

Fig. 10    eine Darstellung einer Magnetisierung.

In der nachfolgenden Beschreibung der bevorzugten Ausführungsbeispiele der vorliegenden Erfindung werden für die in den verschiedenen Zeichnungen dargestellten und ähnlich wirkenden Elemente gleiche oder ähnliche Bezugszeichen verwendet, wobei eine wiederholte Beschreibung dieser Elemente weggelassen wird.

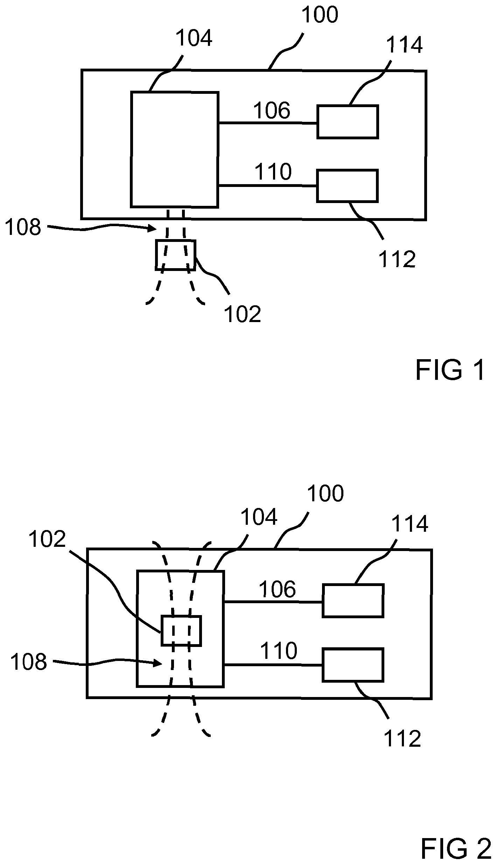

Fig. 1 zeigt eine schematische Darstellung einer Vorrichtung 100 zur Analyse eines magnetische Partikel umfassenden Probenvolumens 102 gemäß einem Ausführungsbeispiel der vorliegenden Erfindung. Die Vorrichtung 100 weist eine Messspule 104 auf. Der in Fig. 1 gezeigte Block 104 kann die Messspule oder eine die Messspule umfassende Schaltung, beispielsweise eine Brückenschaltung repräsentieren. Wird während eines Analysevorganges ein Anregungssignal 106, beispielsweise eine elektrische Spannung oder ein elektrischer Strom über eine Schnittstelle an die Messspule 104 angelegt, so wird von der Messspule 104 ein magnetisches Feld 108 erzeugt.

Die Messspule 104 und das Probenvolumen 102 sind so zueinander ausgerichtet, dass das magnetische Feld 108 auf das Probenvolumen 102 einwirkt, es beispielsweise durchdringt. Das Probenvolumen 102 kann auch als eine Probe aufgefasst werden. Die Induktivität der Messspule 104 ist abhängig von der magnetischen Permeabilität des Probenvolumens 102. Über die Induktivität der Messspule 104 kann somit auf die magnetische Permeabilität des Probenvolumens 102 geschlossen werden. Dazu ist die Messspule 104 mit einer Messschnittstelle zum Erfassen eines von der Induktivität der Messspule 104 abhängigen elektrischen Messsignals 110 auf. Über das Messsignal 110 kann die Induktivität der Messspule 104 und somit die magnetische Permeabilität des Probenvolumens 102 bestimmt werden.

Gemäß diesem Ausführungsbeispiel weist die Vorrichtung 100 eine Auswerteeinrichtung 112 zum Auswerten des elektrischen Messsignals 110 auf. Die Auswerteeinrichtung 112 ist ausgebildet, um das Messsignal 110 zu empfangen und basierend auf dem Messsignal 110 ein Analyseergebnis bezüglich des Probenvolumens 102 zu bestimmen und bereitzustellen. Beispielsweise kann die Auswerteeinrichtung 112 ausgebildet sein, um einen Wert der magnetischen Permeabilität oder einen von der magnetischen Permeabilität des Probenvolumens 102 abhängigen Wert zu bestimmen. Die Messspule 104 und die Auswerteeinrichtung 112 können innerhalb eines gemeinsamen Gehäuses angeordnet sein. Alternativ können die Messspule 104 und die Auswerteeinrichtung 112 in separaten Gehäusen angeordnet sein. Dabei kann die Vorrichtung 100 eine Schnittstelle zum Bereitstellen des Messsignals 110 an eine externe Auswerteeinrichtung 112 aufweisen. Somit kann die Vorrichtung 100 gemäß unterschiedlicher Ausführungsbeispiele sowohl mit als auch ohne die Auswerteeinrichtung 112 realisiert sein.

Gemäß einem Ausführungsbeispiel ist die Auswerteeinrichtung 112 ausgebildet, um die Analyse des Probenvolumens 102 unter Verwendung eines Referenzsignals durchzuführen. Das Referenzsignal kann ein gespeichertes Messsignal repräsentieren, das die Induktivität der Messspule 104 während eines Analysevorganges abbildet, während dem das Probenvolumen 102 einem Referenzvolumen entspricht.

Gemäß einem Ausführungsbeispiel ist die Auswerteeinrichtung 112 ausgebildet, um die Analyse des Probenvolumens 102 unter Verwendung eines Abgleichsignals durchzuführen. Das Abgleichsignal kann einem während des Analysevorganges an die Messspule oder eine die Messspule umfassende Schaltung bereitgestelltes Signal entsprechen.

Gemäß diesem Ausführungsbeispiel weist die Vorrichtung 100 eine Anregungseinrichtung 114 zum Bereitstellen des Anregungssignals 106 auf. Die Anregungseinrichtung 114 kann beispielsweise als eine Stromquelle oder eine Spannungsquelle ausgeführt sein. Die Messspule 104 und die Anregungseinrichtung 114 können innerhalb eines gemeinsamen Gehäuses angeordnet sein. Alternativ können die Messspule 104 und die Anregungseinrichtung 114 in separaten Gehäusen angeordnet sein. Dabei kann die Vorrichtung 100 eine Schnittstelle zum Empfangen des Anregungssignals 106 von der Anregungseinrichtung 114 aufweisen. Somit kann die Vorrichtung 100 gemäß unterschiedlicher Ausführungsbeispiele sowohl mit als auch ohne die Anregungseinrichtung 114 realisiert sein.

Die Vorrichtung 100 kann das Probenvolumen 102, beispielsweise eine Aufnahmeeinrichtung, beispielsweise einen Behälter, für eine Probe umfassen oder das Probenvolumen 102 kann benachbart zu der Vorrichtung 100 angeordnet sein.

Gemäß dem in Fig. 1 gezeigten Ausführungsbeispiel ist das Probenvolumen 102 benachbart zu der Messspule 104, außerhalb der Vorrichtung 100 angeordnet oder anordenbar. Beispielsweise kann das Probenvolumen 102 in Verlängerung einer Längsachse, der beispielsweise als zylindrische Spule ausgeführten Messspule, angeordnet sein.

Zur Analyse des Probenvolumens 102 kann das Probenvolumen 102 zu der Vorrichtung hin bewegt werden, oder umgekehrt.

Gemäß einem Ausführungsbeispiel basiert der Analysevorgang auf der Vermessung der magnetischen Permeabilität der Probe 102 oder des Probenvolumens 102.

Der Parameter in Form der magnetischen Permeabilität ist vorteilhaft, da er eine besonders geeignete Konstruktion der Messapparatur 100 erlaubt.

Gemäß einem Ausführungsbeispiel wird die magnetische Permeabilität der Probe 102 über die Messung der Induktivität der Messspule 104 vermessen. Hierzu wird von der Messspule 104 ein magnetisches Wechselfeld 108 einer bestimmten Stärke erzeugt und nun an dieser Spule 104 entweder Strom oder Spannung vermessen. Daraus lässt sich die magnetische Permeabilität der Probe 102 bei den Feldstärken, die das Wechselfeld 108 durchläuft, bestimmen.

Wird nun lokal, z. B. durch Variation eines Gradienten, entweder die Stärke oder Phase dieses Wechselfeldes 108 variiert, so kann daraus eine lokale Zuordnung der bestimmten magnetischen Permeabilität erfolgen. Dies Variation kann z. B. eine räumlich durchlaufende Welle sein, wo sich die Phase einer sinusförmigen Anregung über den Raum ändert.

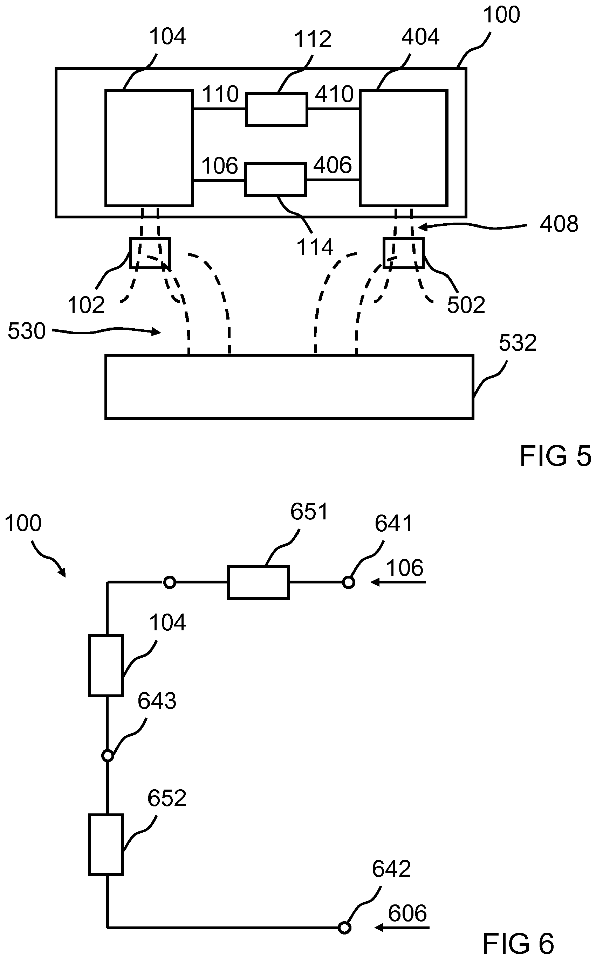

Wird zusätzlich, z. B. durch eine separate Einrichtung, wie es nachfolgend beispielhaft anhand von Fig. 5 gezeigt ist, ein lokal unterschiedliches statisches oder quasistatisches Magnetfeld, so kann aus Variation dessen eine lokale Zuordnung erfolgen. Bei einer Induktionsmessung kann die Ableitung der magnetischen Permeabilität vermessen werden. Für eine lokale Zuordnung ist es gemäß einem Ausführungsbeispiel notwendig, das die magnetische Permeabilität eine Abhängigkeit von der Feldstärke zeigt.

Gemäß einem Ausführungsbeispiel kann das Probenvolumen einem zusätzlichen inhomogenen Magnetfeld ausgesetzt werden. Dies kann für ein Bildgebungsverfahren verwendet werden. Das zusätzliche Magnetfeld wird gemäß einem Ausführungsbeispiel so realisiert, dass es innerhalb des Probenvolumens ein sehr kleines Feld hat, bzw. einen feldfreien Punkt besitzt und an allen anderen Punkten des Probenvolumens groß ist, beispielsweise so groß, dass es eine Sättigung von sich dort befindlichen Partikeln hervorruft. Partikel, die durch das große zusätzliche Magnetfeld gesättigt sind, ändern ihren magnetischen Fluss mit Feldvariationen durch die Messspule 104 nicht wesentlich. Das Messsignal 110 kann daher dem Bereich des Probenvolumens 102 zugeordnet werden, in dem das zusätzliche Magnetfeld die niedrige Feldstärke aufweist. Gemäß einem Ausführungsbeispiel wird unter Verwendung der Messspule 104 fortlaufend die Permeabilität des Probenvolumens 102 vermessen, also Messsignale 110 erfasst, während der feldfreie Punkt des zusätzlichen Magnetfelds mechanisch oder elektrisch verfahren wird. Auf diese Weise kann ein Bild abgerastert werden.

Gemäß einem Ausführungsbeispiel kann somit die räumliche Verteilung magnetischer Partikel in dem Probenvolumen 102 ermittelt werden. Allgemein ausgedrückt kann dazu ein zusätzliches Magnetfeld eingesetzt werden, dessen räumlicher Verlauf seiner magnetischen Feldstärke innerhalb des Probenvolumens 102 einen ersten Abschnitt mit niedriger magnetischer Feldstärke und einen zweiten Abschnitt mit einer im Vergleich zum ersten Abschnitt höheren magnetischen Feldstärke aufweist. Während eines Analysezeitraums wird die räumliche Lage der beiden Abschnitte innerhalb des Probenvolumens 102 verändert. Dies führt zu einer örtlichen Änderung der magnetischen Permeabilität der Partikel innerhalb des Probenvolumens 102. Während des Analysezeitraums erfolgt eine Erfassung einer Mehrzahl von Messsignalen 110, die von der Veränderung der räumlichen Lage der Abschnitte des zusätzlichen magnetischen Felds abhängen. Durch eine Auswertung der Mehrzahl von Messsignalen 110 kann eine Information über die räumliche Verteilung der magnetischen Partikel innerhalb des Probenvolumens 102 ermittelt werden. Die Information über die räumliche Verteilung kann beispielsweise von einem Bildgebungsverfahren genutzt werden, um eine bildliche Darstellung der räumlichen Verteilung der Partikel innerhalb des Probenvolumens 102 zu ermitteln.

Die Information über die räumliche Verteilung kann beispielsweise von der Auswerteeinrichtung 112 bestimmt werden. Dazu kann die Auswerteeinrichtung 112 ausgebildet sein, um eine Information über die Veränderung des zusätzlichen Magnetfelds, beispielsweise eine Information über einen zeitlichen und örtlichen Verlauf der Lage des ersten Abschnitts, beispielsweise eines feldfreien Punkts des zusätzlichen Magnetfelds, mit der Mehrzahl von Messsignalen 110 zu kombinieren. Beispielsweise kann jedem der Mehrzahl von Messsignalen 110 die zum Zeitpunkt der Erfassung eines Messsignals 110 aktuelle Lage des ersten Abschnitts des zusätzlichen Magnetfelds zugeordnet werden. Somit kann jedem der Messsignale 110 eine Position innerhalb des Probenvolumens 102 zugeordnet werden.

Das zusätzliche Magnetfeld kann beispielsweise von einer Einrichtung zum Erzeugen des zusätzlichen Magnetfelds erzeugt werden, wie sie in Fig. 5 gezeigt ist.

Gemäß einem Ausführungsbeispiel kann eine lokale Messung mit einer lokal empfindlichen Spule 104 ebenso zum Aufspüren von Partikeln verwendet werden.

Die magnetische Permeabilität unterscheidet sich von der Magnetisierung, wie sie im Magnetic Particle Imaging (MPI) verwendet wird. So ist es dort in der Regel ausgeschlossen, die Information auf der Anregungsfrequenz zu verwenden. Bei hohen Feldstärken wird dort ebenfalls kein Signal verwendet, da hier die Partikel in Sättigung sind, und demnach die Magnetisierung sich nicht ändert.

Bringt man eine beschriebene Anlage in ein sehr starkes Feld, wie in einen Kernspintomografen, so haben Partikel eine magnetische Permeabilität, die gemessen werden kann, aber die Magnetisierung ändert sich nicht, wenn die Feldstärke variiert wird.

Para- oder Diamagneten ändern zwar ihre Magnetisierung, die Änderung verläuft aber linear und kann dementsprechend auch nicht von einer MPI-Anlage aufgenommen werden, da hier nur nichtlineare Änderungen berücksichtigt werden. Dies kann durch eine Vorrichtung 100 gemäß dem hier beschriebenen Ansatz vermieden werden.

Fig. 2 zeigt eine schematische Darstellung einer Vorrichtung 100 zur Analyse eines magnetische Partikel umfassenden Probenvolumens 102 gemäß einem Ausführungsbeispiel der vorliegenden Erfindung. Die Vorrichtung 100 kann der in Fig. 1 gezeigten Vorrichtung entsprechen, mit dem Unterschied, dass das Probenvolumen 102 gemäß dem in Fig. 2 gezeigten Ausführungsbeispiel im Inneren der Messspule 104 angeordnet oder anordenbar ist, also beispielsweise von einer Mehrzahl von Wicklungen der Messspule 104 umgeben ist.

Zur Analyse des Probenvolumens 102 kann das Probenvolumen 102 in das Innere der Messspule 104 eingeführt werden.

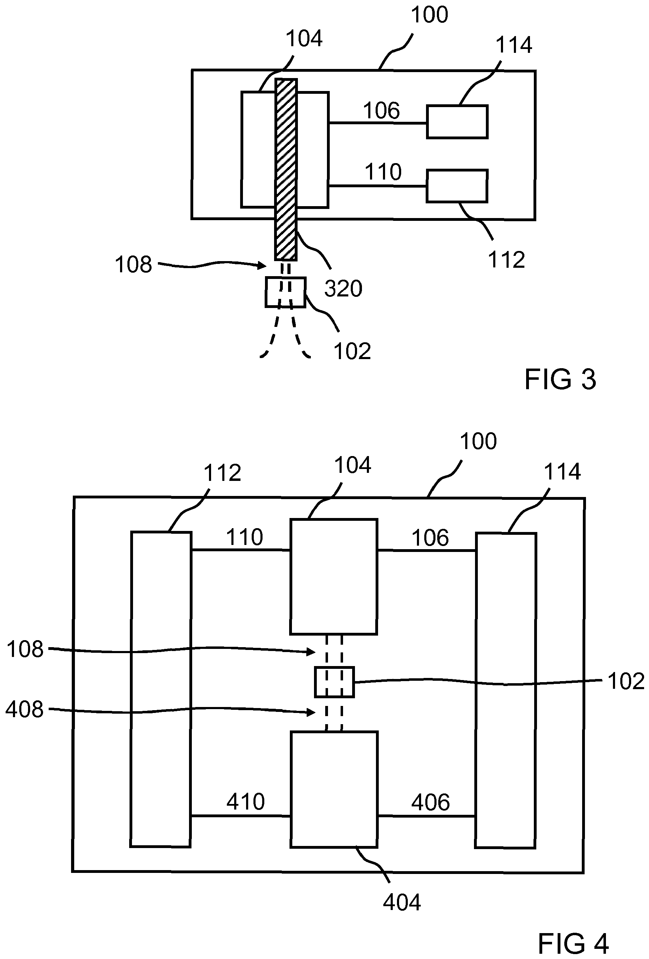

Fig. 3 zeigt eine schematische Darstellung einer Vorrichtung 100 zur Analyse eines magnetische Partikel umfassenden Probenvolumens 102 gemäß einem Ausführungsbeispiel der vorliegenden Erfindung. Die Vorrichtung 100 kann der in Fig. 1 gezeigten Vorrichtung entsprechen, mit dem Unterschied, dass ein Spulenkern 320 durch die Messspule 104 geführt ist, und das Probenvolumen 102 gemäß dem in Fig. 3 gezeigten Ausführungsbeispiel an einem Ende des Spulenkerns 320 angeordnet ist oder anordenbar ist.

Fig. 4 zeigt eine schematische Darstellung einer Vorrichtung 100 zur Analyse eines magnetische Partikel umfassenden Probenvolumens 102 gemäß einem Ausführungsbeispiel der vorliegenden Erfindung. Die Vorrichtung 100 kann der in Fig. 1 gezeigten Vorrichtung entsprechen, mit dem Unterschied, dass die Vorrichtung 100 eine weitere Messspule 404 aufweist und das Probenvolumen 102 zwischen den beiden Messspulen 104, 404 angeordnet ist oder anordenbar ist. Beispielsweise können die Messspulen 104, 404 eine gemeinsame Längsachse aufweisen, die durch das Probenvolumen 102 führt.

Wird ein weiteres Anregungssignal 406 an die weitere Messspule 404 angelegt, so wird von der weiteren Messspule 404 ein weiteres magnetisches Feld 408 erzeugt. Die weitere Messspule 404 und das Probenvolumen 102 sind so zueinander ausgerichtet, dass das weitere magnetische Feld 408 auf das Probenvolumen 102 einwirkt, es beispielsweise durchdringt. Die Induktivität der weiteren Messspule 404 ist abhängig von der magnetischen Permeabilität des Probenvolumens 102. Über die Induktivität der weiteren Messspule 404 kann somit auf die magnetische Permeabilität des Probenvolumens 102 geschlossen werden. Dazu weist die Vorrichtung 100 eine weitere Messschnittstelle zum Erfassen eines von der Induktivität der weiteren Messspule 404 abhängigen weiteren elektrischen Messsignals 410 auf. Über das weitere Messsignal 410 kann die Induktivität der weiteren Messspule 404 und somit die magnetische Permeabilität des Probenvolumens 102 bestimmt werden.

Gemäß diesem Ausführungsbeispiel ist die Auswerteeinrichtung 112, die wiederum in Bezug auf die Vorrichtung 100 als interne oder externe Einrichtung realisiert sein kann, ausgebildet, um die beiden elektrischen Messsignals 110, 410 zu erfassen und zur Analyse des Probenvolumens 102 auszuwerten. Zum Auswerten der Messsignale 110, 410 kann die Auswerteeinrichtung 112 zusätzlich eine Information über die Anregungssignale 106, 406 empfangen und zum Auswerten der Messsignale 110 verwenden.

Die Anregungseinrichtung 114 ist ausgebildet, um die Anregungssignale 106, 406 zu erzeugen und an die Messspulen 104, 404 bereitzustellen. Die Anregungseinrichtung 114 ist gemäß einem Ausführungsbeispiel ausgebildet, um die Anregungssignale 106, 406 mit unterschiedlicher Charakteristik, beispielsweise unterschiedlicher Phase, bereitzustellen. Eine Information über die Charakteristika der Anregungssingale 106, 406 kann über eine Schnittstelle von der Anregungseinrichtung 114 an die Auswerteeinrichtung 112 bereitgestellt werden.

Fig. 5 zeigt eine schematische Darstellung einer Vorrichtung 100 zur Analyse eines magnetische Partikel umfassenden Probenvolumens 102 gemäß einem Ausführungsbeispiel der vorliegenden Erfindung. Die Vorrichtung 100 kann der in Fig. 4 gezeigten Vorrichtung entsprechen, mit dem Unterschied, dass die Messspulen 104, 404 gemäß diesem Ausführungsbeispiel nebeneinander angeordnet sind, also beispielsweise Längsachsen der Messspulen 104, 404 parallel zueinander verlaufen. Die weitere Messspule 404 wird gemäß diesem Ausführungsbeispiel verwendet, um ein weiteres Probenvolumen 502 zu analysieren. Alternativ können die Messspulen 104, 404 verwendet werden, um ein gemeinsames Probenvolumen zu analysieren, dass die gezeigten Probenvolumen 102, 502 umfasst.

Die Auswerteeinrichtung 112 kann ausgebildet sein, um die Probenvolumen 102, 502 separat auszuwerten oder um eine räumliche Verteilung magnetischer Partikel innerhalb des gemeinsamen Probenvolumens zu erzeugen. Beispielsweise kann die Auswerteeinrichtung 112 ausgebildet sein, um eine bildliche Darstellung der räumlichen Verteilung der magnetischen Partikel oder einer Darstellung der magnetischen Permeabilität innerhalb des gemeinsamen Probenvolumens 102, 502 zu erstellen.

Zur Analyse der Probenvolumen 102, 502 kann die Auswerteeinrichtung 112 eine Information über Charakteristika der Anregungssignale 106, 406 sowie eine Information über Positionen, beispielsweise Relativpositionen, der Probenvolumen 102, 502 oder der Messspulen 104, 404 verwenden.

Zusätzlich oder alternativ kann die Auswerteeinrichtung 112 eine Information über eine Charakteristik eines zusätzlichen Magnetfelds 530 empfangen und zur Analyse der Probenvolumen 102, 505 verwenden. Das zusätzliche Magnetfeld 530 wird gemäß diesem Ausführungsbeispiel von einer Einrichtung 532 zum Erzeugen des zusätzlichen Magnetfelds 530 erzeugt, beispielsweise von einer Anordnung aus einem oder mehreren Magneten oder einer oder mehreren Spulen erzeugt. Die Einrichtung 532 kann auch Teil der Vorrichtung 100 sein. Das zusätzliche Magnetfeld 530 kann beispielsweise wie anhand von Fig. 1 beschrieben ausgeführt und eingesetzt werden.

Fig. 6 zeigt eine schematische Darstellung einer Vorrichtung 100 zur Analyse eines magnetische Partikel umfassenden Probenvolumens gemäß einem Ausführungsbeispiel der vorliegenden Erfindung. Gezeigt ist eine mögliche Verschaltung der Messspule 104, der Vorrichtung 100. Eine solche Verschaltung kann auch in den in den vorangegangenen Figuren gezeigten Vorrichtungen 100 eingesetzt werden.

Die Vorrichtung 100 weist einen ersten Anschluss 641, einen zweiten Anschluss 642 und eine Messschnittstelle 643 auf.

Ein erster Kontakt der Messspule 104 ist über ein erstes Element 651, beispielsweise einen Kondensator, mit dem ersten Anschluss 641 verbunden. Ein zweiter Kontakt der Messspule 104 ist mit der Messschnittstelle 643 und über ein zweites Element 652, beispielsweise einen Widerstand, mit dem zweiten Anschluss 642 verbunden.

Über den ersten Anschluss 641 kann ein Anregungssignal 106, wie anhand der vorangegangenen Figuren beschrieben, eingespeist werden. Dazu kann der erste Anschluss 641 mit einer geeigneten Anregungsschaltung, wie beispielsweise anhand von Fig. 1 beschrieben, verbunden sein.

Über den zweiten Anschluss 642 kann ein Abgleichsignal 606 eingespeist werden. Dazu kann der erste Anschluss 641 beispielsweise ebenfalls mit der Anregungsschaltung verbunden sein, die dann ausgebildet ist, um neben dem Anregungssignal 106 auch das Abgleichsignal 606 bereitzustellen.

Gemäß einem Ausführungsbeispiel liegt während eines Analysevorganges der Vorrichtung 100 ein Messsignal an der Messschnittstelle 643 an, wie es bereits beschrieben ist. Das Messsignal kann beispielsweise von einer Auswerteeinrichtung 112 erfasst und ausgewertet werden, um das Probenvolumen zu analysieren.

Gemäß einem weiteren Ausführungsbeispiel wird während des Analysevorganges das Abgleichsignal 606 so in den zweiten Anschluss 642 eingespeist, dass an der Messschnittstelle 643 ein vorbestimmtes gleichbleibendes Referenzsignal anliegt. Dazu kann das Abgleichsignal 606 beispielsweise von einer Regeleinrichtung unter Verwendung des an der Messschnittstelle 643 anliegenden Signals so geregelt werden, dass an der Messschnittstelle 643 das Referenzsignal anliegt. Das sich daraus ergebende Abgleichsignal kann dann von der Auswerteeinrichtung 112 als Messsignal ausgewertet werden, um das Probenvolumen zu analysieren.

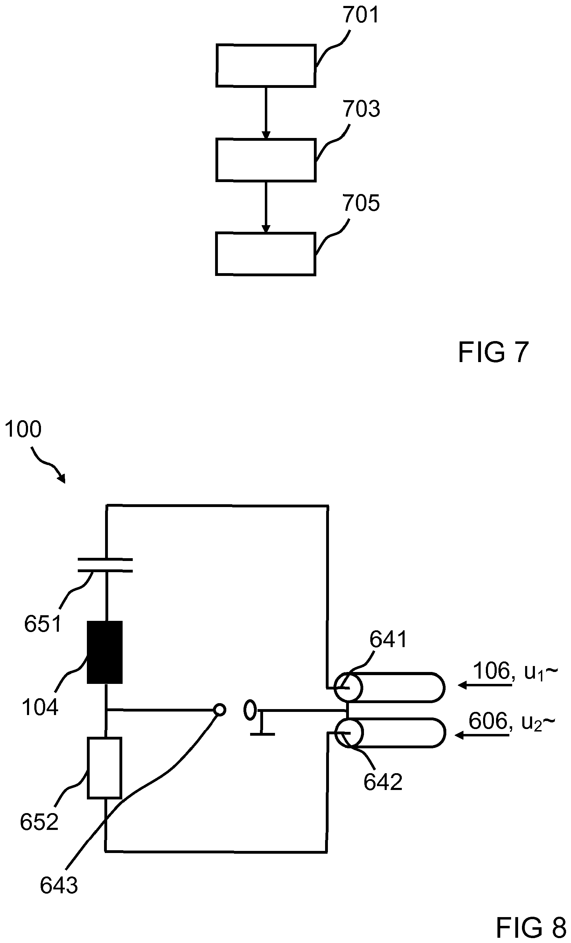

Fig. 7 zeigt ein Ablaufdiagramm eines Verfahrens zur Analyse eines magnetische Partikel umfassenden Probenvolumens gemäß einem Ausführungsbeispiel der vorliegenden Erfindung. Das Verfahren kann beispielsweise im Zusammenhang mit einer Vorrichtung angewendet werden, wie sie anhand der vorangegangenen Figuren beschrieben ist.

In einem Schritt 701 wird ein elektrisches Anregungssignal an eine Messspule angelegt. Angeregt durch das Anregungssignal ist die Messspule ausgebildet, um ein auf das Probenvolumen einwirkendes magnetisches Feld zu erzeugen. In einem Schritt 703 wird ein elektrisches Messsignal erfasst, das von der Induktivität der Messspule abhängig ist. In einem Schritt 705 wird basierend auf dem Messsignal eine magnetische Permeabilität des Probenvolumens analysiert.

Gemäß einem Ausführungsbeispiel kann das Messsignal im Schritt 705 unter Verwendung eines Referenzsignals analysiert werden. Das Referenzsignal kann ermittelt werden, indem die Schritte 701, 703 des Verfahrens durchgeführt werden, während das magnetische Feld der Messspule auf ein Referenzvolumen wirkt. Das Referenzsignal oder ein dem Referenzsignal entsprechender Wert kann beispielsweise nach seiner Bestimmung in einer Speichereinrichtung gespeichert und aus der Speichereinrichtung ausgelesen werden.

Beispielsweise kann ein solches Verfahren zur Analyse eines magnetische Partikel umfassenden Probenvolumens gemäß einem Ausführungsbeispiel der vorliegenden Erfindung im Zusammenhang mit einer Vorrichtung angewendet werden, wie sie anhand von Fig. 6 beschrieben ist.

Dabei wird im Schritt 701 das Anregungssignal an den ersten Anschluss der Vorrichtung und das Abgleichsignal wird an den zweiten Anschluss der Vorrichtung angelegt. Im Schritt 703 wird das an der Messschnittstelle anliegende Signal als Messsignal erfasst.

Das im Schritt 701 anzulegende Abgleichsignal kann dabei im Voraus ermittelt und gespeichert werden, indem die Schritte 701, 703 des Verfahrens durchgeführt werden, während das magnetische Feld der Messspule auf ein Referenzvolumen wirkt. Dabei wird das Abgleichsignal so eingestellt, dass an der Messschnittstelle ein Referenzsignal anliegt.

Werden die Schritte des Verfahrens ausgeführt, während das magnetische Feld der Messspule auf das Probenvolumen einwirkt, so unterscheidet sich das Messsignal trotz unverändertem Anregungssignal und Abgleichsignal von dem Referenzsignal, sofern sich die magnetische Permeabilität des Probenvolumens von dem Referenzvolumen unterscheidet. Aus der Differenz zwischen dem Referenzsignal und dem Messsignal und unter Kenntnis der magnetischen Permeabilität des Referenzvolumens kann somit auf die magnetische Permeabilität des Probenvolumens geschlossen werden.

Gemäß einem weiteren Ausführungsbeispiel wird im Schritt 701 das Anregungssignal an den ersten Anschluss der Vorrichtung und das Abgleichsignal wird an den zweiten Anschluss der Vorrichtung angelegt. Dabei wird das Abgleichsignal so eingestellt, dass sich an der Messschnittstelle ein Referenzsignal einstellt. Bei Analysevorgängen von unterschiedlichen Probenvolumen wird das Abgleichsignal jeweils so eingestellt, dass das Referenzsignal an der Messschnittstelle anliegt.

Werden die Schritte des Verfahrens ausgeführt, während das magnetische Feld der Messspule auf das Probenvolumen einwirkt, so unterscheidet sich das Abgleichsignal trotz unverändertem Anregungssignal von dem bei einem Referenzvolumen eingestellten Abgleichsignal, sofern sich die magnetische Permeabilität des Probenvolumens von dem Referenzvolumen unterscheidet. Aus der Abweichung zwischen den Abgleichsignalen, die bei dem Referenzvolumen und dem Probenvolumen eingestellt werden, kann unter Kenntnis der magnetischen Permeabilität des Referenzvolumens auf die magnetische Permeabilität des Probenvolumens geschlossen werden. Das bei dem Referenzvolumen eingestellte Abgleichsignal oder ein diesem Abgleichsignal entsprechender Wert kann in einer Speichereinrichtung gespeichert und im Schritt 705 des Analysierens aus der Speichereinrichtung ausgelesen werden.

Gemäß einem weiteren Ausführungsbeispiel kann das Verfahren unter Verwendung von zwei oder mehr Messspulen angewendet werden, um ein oder mehrere Probenvolumen zu analysieren.

In dem Schritt 701 wird dazu ein elektrisches Anregungssignal an eine Messspule und mindestens ein weiteres elektrisches Anregungssignal an mindestens eine weitere Messspule bereitgestellt. In dem Schritt 703 werden von den Induktivitäten der Messspulen abhängige Messsignale erfasst und im Schritt 705 ausgewertet, um das Probenvolumen oder die Probenvolumen zu analysieren.

Im Schritt 705 können zum Auswerten der Messsignale Informationen über Charakteristika der Anregungssignale und zusätzlich oder alternativ Charakteristika über ein oder mehrere zusätzliche auf das oder die Probenvolumen einwirkenden Magnetfelder einfließen. Dabei kann im Schritt 705 eine räumliche Verteilung von magnetischen Partikeln oder Werten der magnetischen Permeabilität innerhalb des oder der Probenvolumen ermittelt werden.

Fig. 8 zeigt eine schematische Darstellung einer Vorrichtung 100 zur Analyse eines magnetische Partikel umfassenden Probenvolumens gemäß einem Ausführungsbeispiel der vorliegenden Erfindung. Gezeigt ist eine mögliche Verschaltung der Messspule 104, der Vorrichtung 100. Eine solche Verschaltung kann auch in den in den vorangegangenen Figuren gezeigten Vorrichtungen 100 eingesetzt werden.

Die Vorrichtung 100 weist einen ersten Anschluss 641, einen zweiten Anschluss 642 und eine Messschnittstelle 643 auf.

Ein erster Kontakt der Messspule (L) 104 ist über ein erstes Element 651, hier einen Kondensator (C), mit dem ersten Anschluss 641 verbunden. Ein zweiter Kontakt der Messspule 104 ist über eine Leitung mit der Messschnittstelle 643 und über ein zweites Element 652, hier einen Widerstand (R), mit dem zweiten Anschluss 642 verbunden.

Über den ersten Anschluss 641 kann beispielsweise über eine erste Koaxialleitung ein Anregungssignal 106, hier in Form einer ersten Wechselspannung U1~ zugeführt werden. Über den zweiten Anschluss 642 kann beispielsweise über eine zweite Koaxialleitung ein Abgleichsignal 606, hier in Form einer zweiten Wechselspannung U2~ zugeführt werden.

Je nach Ausführungsbeispiel können zwischen der Messschnittstelle 643 und einem Masseanschluss 845 der Vorrichtung 100 das bereits beschriebene Messsignal oder das bereits beschriebene Referenzsignal abgegriffen werden.

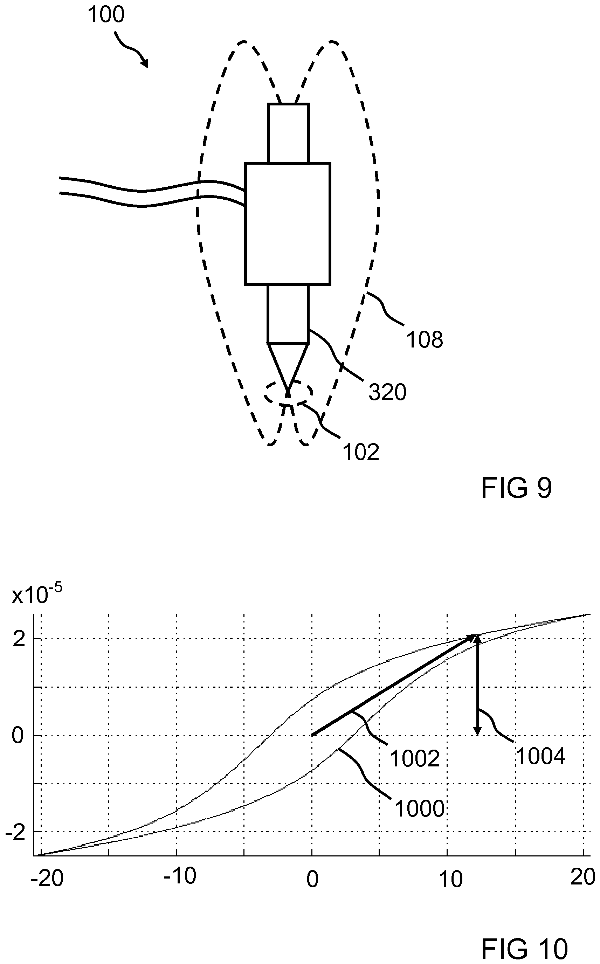

Fig. 9 zeigt eine schematische Darstellung einer Vorrichtung 100 zur Analyse eines magnetische Partikel umfassenden Probenvolumens gemäß einem Ausführungsbeispiel der vorliegenden Erfindung. Die Vorrichtung 100 weist eine Messspule mit einem Spulenkern 320 auf, wie es beispielsweise anhand von Fig. 3 beschrieben ist. Der Spulenkern 320 ist als ein zylinderförmiger Stab ausgeführt, der an einem Ende ein flaches Ende und an dem anderen Ende eine Spitze aufweist. Die Spitze ist in ein Probenvolumen 102 hingeführt oder auf das Probenvolumen 102 gerichtet. Durch die Spitze des Spulenkerns 320 werden die Magnetfeldlinien des magnetischen Felds 108 auf das Probenvolumen 102 konzentriert. Die Vorrichtung 100 weist als eine Schnittstelle beispielsweise zwei elektrische Leitungen zur Energieversorgung oder zum Zuführen oder Auslesen von Signalen auf.

Anhand der vorangegangenen Figuren werden im Folgenden unterschiedliche Ausführungsbeispiele der vorliegenden Erfindung beschrieben.

Den Ausführungsbeispielen zugrunde liegend ist eine Messung der magnetischen Permeabilität.

Die magnetische Permeabilität ist als die Eigenschaft magnetisches Feld in Materie zu leiten direkt über die Induktivität der Wechselfeld-erzeugenden Spule 104 zugänglich. Dies ermöglicht einen vorteilhaften Bau einer solchen Messeinrichtung 100. Im einfachsten Falle besteht diese aus einer einzelnen Spule 104, die bei Anlegen einer Wechselspannung 106 in einem Messvolumen 102 ein Magnetfeld 108 einer bestimmten Stärke erzeugt. Die Induktivität der Spule 104 hängt nun direkt von der magnetischen Permeabilität ab und kann durch eine einfache Strom- oder Spannungsmessung, beispielsweise über das Messsignal 110, bestimmt werden. Hier ist es durch geeignetes elektrisches Verschalten, wie eine Brückenschaltung in Kombination mit einer Spannungsmessung, leicht möglich das gemessene Spannungssignal 110 so einzustellen, dass die gemessene Spannung 110 nur der Änderung der Spannung 110 entspricht, die sich durch Einbringen einer Probe in das Probenvolumen 102 und damit durch Änderung der magnetischen Permeabilität, ergibt. Damit kann der Dynamikbereich eines Spannungsmessers der Spannung 110 für die relevante Information benutzt werden.

Eine solche Messbrücke besteht gemäß einem Ausführungsbeispiel aus einer Spule 104, die Feld im Probenvolumen 102 erzeugen kann, und einem elektrischen Widerstand 652. Die über das Anregungssignal 106 angelegte Spannung an der Messspule 104 wird so gewählt, dass dort das gewählte magnetische Feld 108 erzeugt wird. Die über das Abgleichsignal 606 angelegte Spannung an dem Brückenwiderstand 652 wird entsprechend so gewählt, dass in einfachsten Fall ohne Probe am Spannungsmesser keine Spannung 110 abfällt. Bringt man nun eine Probe in das Volumen 102 der Messspule 104, so kann aus dem Spannungsverlauf 110 die magnetische Permeabilität der Probe in Abhängigkeit der angelegten Magnetfeldstärke des Felds 108 bestimmt werden. Daraus kann auf das Vorhandensein und die Eigenschaften, beispielsweise die Stoffmenge, des magnetischen Materials in dem Probenvolumen 102 geschlossen werden.

Gemäß einem Ausführungsbeispiel wird die Spannung U1~ 106 so gewählt, dass in der Spule 104 ein geeignetes Wechsel-Magnetfeld 108 erzeugt wird. Der in Fig. 8 gezeigte Kondensator 651 ist prinzipiell nicht notwendig, aber eine gute Option um den Blindwiderstand zu reduzieren, damit bei geringer Spannung ein großer Strom erzeugt werden kann. Als Anregungssignal 106 ist im speziellen eine sinusförmige Spannung auf einer Frequenz, z. B. 20kHz, geeignet. Das Feld 108, das dadurch erzeugt wird, ist idealerweise größer als 5mT an der Spitze, und kann bis zu einigen 100 mT gehen. Die Spannung U2~ 606 wird in ihrem zeitlichen Verlauf so gewählt, dass bei der leeren Messspule 104, also leerem Probenvolumen 102, gerade am Ausgang 643, also der Spannung zwischen Masse und dem Verbindungspunkt von Spule 104 und Widerstand 652 immer als Referenzsignal eine Spannung von 0V erzeugt wird. Im einfachsten Fall ist die Abgleichspannung 606 eine ebenso sinusförmige Spannung eines bestimmten Pegels und einer bestimmten Phase gegenüber der Spannung U1~ 106. Wird nun eine magnetisierbare Probe in die Spule 104 eingebracht, oder in die Nähe der Spule 104 gebracht, so ergibt sich ein Spannungsverlauf am Ausgang 643, der abhängig von der magnetischen Permeabilität der Probe in dem Probenvolumen 102 ist.

Eine Messbrücke, wie sie beispielsweise in Fig. 8 gezeigt ist, hat zwei wesentliche Betriebsmodi, den Brückenabgleich und die Messung der Brückenspannung.

Bei Brückenabgleich wir die zeitabhängige Spannung am Widerstand so eingestellt, dass die Brückenspannung an der Messschnittstelle 643 immer Null wird. Das eigentliche Messsignal 110 ist nun die Differenz zwischen der Spannung am Widerstand 652 mit und ohne Probe in dem Probenvolumen 102.

Beim Messen der Brückenspannung wird die Spannung am Widerstand 652 so eingestellt, dass bei leerer Messspule 104, also leerem Probenvolumen 102, die Brückenspannung an der Messschnittstelle 643 zu Null wird. Nun ist die gemessene Brückenspannung das eigentliche Messsignal 110.

Es ist auch möglich, eine Kombination dieser beiden Modi zu verwenden, und so als Dynamikbereich eine Summe des Dynamikbereichs des Spannungsmessers und der Spannung am Widerstand 652 zu erhalten.

Diese Messanordnung kann auch in anderen Ausführungsformen realisiert werden. So kann statt einer Spannungsmessung auch eine Strommessung erfolgen.

Vorteilhaft vor allem bei großen Messvolumina 102 ist eine resonante Anpassung der Messspule 104 auf die Frequenz des Wechselfeldes 108, so kann mit geringerer Spannung gearbeitet werden. Gemäß einem Ausführungsbeispiel wird die Spule 104 mit einem geeignet gewählten Kondensator 651 in Reihe. Ebenso kann der in Fig. 8 gezeigte Brückenwiderstand 652 durch eine komplexere Anordnung von Spulen, Kondensatoren und Widerständen ersetzt werden. Vorteilhaft ist es in einigen Fällen zur Unterdrückung von Störungen aus dem umgebenden Raum, hier eine der Messspule 104 gleichartige Spule mit gegensätzlicher Feldrichtung zu wählen. Diese Anordnung wirkt wie ein Gradiometer, ein in beiden Spulen gleichermaßen erzeugtes Spannungssignal hebt sich auf. Der Messspule 104 können auch komplexere Anordnungen elektrischer Bauteile parallel oder in Reihe geschaltet werden, um die Eigenschaften wie Resonanzen anzupassen.

Eine besondere Ausformung eines solchen Messsystems 100 ist eine Spule 104, die ein Messvolumen 102 umschließt und dort ein homogenes Wechselfeld 108 erzeugt. Ebenso eine besondere Anordnung ist eine Messspule 104, die vor einer Oberfläche ein homogenes oder linear in der Stärke mit dem Abstand von der Oberfläche abfallendes Magnetfeld 108 erzeugen kann.

Es ist einsichtig, das sich diese Messapparatur 100 in besonderer Weise zur Messung auf der Anregungsfrequenz eignet, eine resonante Abstimmung macht diese auf genau dieser Frequenz besonders empfindlich. Dies ist aber kein Nachteil, da zum einen dies eine wertvolle Information ist, und zum Zweiten führen vor allem Nichtlinearitäten der magnetischen Permeabilität zu einer Reihe höherer Harmonischer, für die dann eine breitbandige Messung erforderlich ist. Dadurch ist eine resonante Abstimmung auf eine andere Frequenz nur eingeschränkt sinnvoll. Dennoch ist es nicht erforderlich, die Messspule 104 resonant auf die Anregungsfrequenz abzustimmen. Stattdesen kann die Abstimmung prinzipiell auch auf einer anderen Frequenz erfolgen, auch stehen eine Reihe bekannter Ansätze zur mehrfachresonanten Abstimmung von Spulen zur Verfügung.

Eine Lokalisierung der Partikel innerhalb des Probenvolumens 102 erfolgt durch einen oder eine Kombination folgender Mechanismen.

Ist die Spule 104 nur lokal empfindlich, so kann hierüber eine lokale Bestimmung der Partikel erfolgen. Hierbei ist zu bemerken, dass diese Konstruktion sich einfach miniaturisieren lässt und so z. B. in einen Katheder integrieren lässt. So kann z. B. in Blutgefäßen so eine lokale Messung magnetischer Partikel erfolgen.

Eine besondere Erweiterung ist die Möglichkeit, in diese Messanordnung 100 zur Leitung des Feldes 108 an einen anderen Ort, einen oder mehrere magnetisch leitende Kerne 320 zu verwenden. So ist es zum Beispiel möglich, eine Nadel aus einem ferromagnetischen Material in die Spule 104 einzusetzen, wie es in Fig. 9 gezeigt ist. Die Spule 104 erzeugt nun in dem Kern 320 einen moderaten magnetischen Fluss, der unterhalb der Sättigung gehalten werden kann, an der Nadelspitze aber einen hohen Fluss. Magnetisierbare Partikel, die sich im Bereich 102 der Nadelspitze befinden, erzeugen nun ein besonders hohes Antwortsignal, da hier die Magnetisierung am größten ist. Damit ist ein Mikroskop realisierbar.

Beispielsweise erzeugt die in Fig. 9 gezeigte Anordnung ein hohes lokales Feld im angedeuteten Bereich, der als Probenvolumen 102 dienen kann. Eine ergänzend einsetzbare weitere Messanordnung ist dabei nicht abgebildet.

Eine Lokalisierung von Partikel mit einer Vorrichtung 100, wie sie anhand der Figuren beschrieben ist, kann über eine Arrayanordnung von einzelnen Messspulen 104, 404 erfolgen. Hierbei kann die Arrayanordnung im einfachsten Falle, z. B. bei einer zweidimensionalen Messung an einer Objektoberfläche, so erfolgen, dass die einzelnen Elemente mit den einzelnen Messspulen 104, 404 eigene angegrenzte Messvolumina 102, 502 messen, und daraus ein Bild berechnet wird.

Gemäß einem Ausführungsbeispiel können die einzelnen Elemente mit den einzelnen Messspulen 104, 404 so angeordnet werden, dass die einzelne Messung in überlappenden Volumina 104, 404 stattfindet. Hier kann durch einen geeigneten Feldverlauf der Felder 108, 408 der Einzelspulen 104, 404, wie homogene Anregungsfelder oder Anregungsfeldgradienten in Kombination mit einer individuellen oder gleichen Frequenz, Amplitude und Phase der Anregungsspannung, ein lokal unterschiedliches zeitabhängiges Magnetfeld ausgebildet werden, das sich in Amplitude und/oder Phase unterscheidet. Durch eine geeignete Kombination von Anregungsmustern kann daraus ein Bild berechnet werden. Ein einfaches Beispiel zur Verdeutlichung sind z. B. zwei gegenüberliegende Spulen 104, 404, wie es beispielsweise in Fig. 4 gezeigt ist, deren Magnetfeld 108, 408 durch die Konstruktion der Spulen 104, 404 im Messvolumen 102 dazwischen so gewählt wird, das, werden die beiden Spulen 104, 404 mit unterschiedlicher Phase der Wechselspannung angesteuert, die Amplitude des Gesamtmagnetfeldes annähernd gleich ist, sich daraus aber eine unterschiedliche Phase ergibt, je nachdem an welcher Spule 104, 404 man näher ist. Die magnetische Permeabilität (im Allgemeinen ein komplexer Wert) einer Punktprobe zeigt über ihre Phase nun (hier eindimensional) den Ort zwischen den Spulen 104, 404.

Solche Anordnungen lassen sich je nach Messobjekt individuell optimieren, wie das lineare Aufreihen von Leiterschleifen für zylindrische Objekte für eine eindimensionale Messung. Mit mehreren solcher gegenüberliegenden Spulen 104, 404 in einer dreidimensionalen Anordnung können entsprechend dreidimensionale Kodierungen des Ortes vorgenommen werden.

Es besteht weiterhin die Möglichkeit, durch zusätzliche Magnetfelder 530, wie extern erzeugte Magnetfeldgradienten, im einfachsten Fall durch Anti-Helmholtz-Spulen, die magnetische Permeabilität zu ändern, und damit lokal zu kodieren. Eine solche Anordnung ist beispielsweise in Fig. 5 gezeigt. In diesem Fall kann die zur Magnetisierung sehr ähnliche nichtlineare magnetische Permeabilität gemessen und zur Bildrekonstruktion verwendet werden.

Die magnetische Permeabilität in starken Magnetfeldern, wie die eines MRT, ist eine messbare Größe und kann auch hier zur Partikeldetektion und zur Charakterisierung herangezogen werden.

In einem einfachen Beispiel kann eine Lokalisation und Charakterisation der Partikel durch eine lokale empfindliche Spule, wie ein Katheder mit integrierter Messpule 104, lokal die Anwesenheit von Partikeln messen. Als Beispiel lässt sich so die Anwesenheit eines paramagnetischen Stoffes wie Dysprosium-III-Oxyd bestimmen. Ebenso lassen sich aber auch die anderen vorgestellten Techniken umsetzen.

Gemäß einem Ausführungsbeispiel ist die gezeigte Vorrichtung 100 als ein Spektrometer für die Detektion magnetischer Partikel, die sich in einem entsprechenden Probenvolumen 102 befinden können, ausgeführt. Die Besonderheit ist hier, dass nur eine einzige Spule 104 zur Erzeugung eines Anregungs- und Detektionsfeldes 108 benötigt wird. Aufgrund der besonderen elektrischen Verschaltung der Spule 104 ist es möglich, das Signal der magnetischen Partikel ohne besondere Vorkehrungen in der Konstruktion der Spule 104 hintergrundfrei zu erhalten.

Dabei ist zu beachten, dass diese Anlage für eine Signalaufnahme auf der Anregungsfrequenz der Spule 104 besonders geeignet ist. Damit ist es möglich, auch Partikel mit linearer Magnetisierung in Abhängigkeit der Feldstärke, wie es üblicherweise Diamagneten und Paramagneten zeigen, zu vermessen.

Ein solches Spektrometer kann sowohl einzeln als Spektrometer für magnetische Partikel betrieben werden, als auch als Teil einer bildgebenden Anlage. Ebenso sind statische Magnetfelder 530 als Offset für die Messung in der Anlage realisierbar, ebenso wie das Zusammenwirken mit extern erzeugten statischen magnetischen Offset-Feldern 530.

Die magnetische Permeabilität von magnetischen Nanopartikeln gibt wichtige Aufschlüsse über die Partikel selber, aber auch über Ihre Umgebung. So ist es möglich Konzentrationen von Partikeln zu bestimmen, aber auch Eigenschaften der Partikel wie Größe oder Temperatur. Auch die Umgebung hat einen Einfluss, so kann z. B. die Viskosität des umgebenden Mediums einen Einfluss auf das Signal haben und so vermessen werden.

Bei geeigneter Wahl der Partikel, wie z. B. Partikel mit einer nichtlinearen Magnetisierungskurve, d. h. die Magnetisierung und demzufolge die magnetische Permeabilität der Partikel steigt nichtlinear mit einem angelegten Magnetfeld, und einem ortvariablen Magnetfeld 530 kann eine lokale Zuordnung des Signals erfolgen und so ein Bild generiert werden.

Insbesondere ist eine Lokalisation von magnetisierbaren Substanzen wie Eisenoxydpartikeln oder paramagnetischen Substanzen möglich.

Dabei kann eine Miniaturisierung einer Messsonde für solche Substanzen durchgeführt werden, z. B. für einen Katheder.

Auch ist eine Detektion solcher Substanzen in starken Magnetfeldern möglich, wie sie z. B. im MRT vorliegen. Insbesondere ist auch eine Lokalisation solcher Substanzen in starken Magnetfeldern 530 realisierbar..

Gemäß einem Ausführungsbeispiel wird eine magnetisierbare Probe 102 in die Spule 104 eingebracht und der Spannungsverlauf an der als Ausgang dienenden Messschnittstelle 643 wird ausgewertet. Der Spannungsverlauf an der Messschnittstelle 643 ist dabei abhängig von der magnetischen Permeabilität der Probe 102.

Entsprechend kann auch der Strom in der Spule 104 vermessen werden. Der Strom ist ebenfalls abhängig von der magnetischen Permeabilität der Probe 102.

Eine solche Messung kann zur Lokalisation von Partikeln verwendet werden.

Dies kann durch eine Miniaturisierung des Messaufbaus 100 auch in Verbindung mit Messarrays oder Messfeldern geschehen.

Es besteht die Möglichkeit, eine Lokalisierung durch Verwendung eines nadelförmigen magnetisch leitfähigen Kerns 320 zu erzielen. Hier wird an der Nadelspitze des Kerns 320 lokal ein großes Magnetfeld 108 erzeugt, in dem der Nachweis der Partikel stattfinden kann.

Die Lokalisierung kann durch räumlich variable Amplituden und/oder Phasenänderung des erzeugten Messmagnetfeldes 108 erzeugt werden.

Es können lokale variable statische oder quasistatische zusätzliche Magnetfelder 530 verwendet werden.

Die Funktionsweise ist dabei ähnlich wie das Magnetic Particle Imaging (MPI) mit dem Unterschied, das hier ein physikalisch anderer Parameter der Probe gemessen wird. Die Magnetisierung lässt sich dabei aus der Permeabiliät berechnen. Es ist einfacher und messtechnisch vorteilhaft, die magnetische Permeabilität zu vermessen. Damit können auch para- und diamagnetische Substanzen vermessen werden. Ferner ist es möglich, die Messung auch in starken magnetischen Feldern 530, wie in MR-Tomografen, durchzuführen.

Die MPI vermisst die Änderung der Magnetisierung M, das vorgestellte Verfahren die magnetische Permeabilität.

Fig. 10 zeigt eine Magnetisierung-Kurve von superparamagnetischen Nanopartikeln als Suspension. Die magnetische Permeabilität ist dabei wie folgt bestimmt:

Magnetische Permeabilität = ( (Magnetisierung)/(Feldstärke) +1)

Auf der X-Achse ist die Feldstärke in mT und auf der Y-Achse die Magnetisierung in Am$^2$ aufgetragen. Gezeigt ist die Magnetisierungskurve 1000 der Partikel. Der Pfeil 1002 symbolisiert dabei so etwas wie magnetische Suszeptibilität (M/H) der Y-Achsenabschnitt 1004 etwa die Magnetisierung M (hier für etwa 12mT Anregungsfeld).

Der beschriebene Ansatz kann in unterschiedlichen Ausführungsbeispielen realisiert werden. Zum einen ist eine Miniaturisierung der Anordnung 100 zur lokalen Bestimmung möglich. Zum anderen kann eine Arrayanordnung 100 realisiert werden, wie linear aufgereiht um die Probe 102 angebrachte Spulen 104, 404, mit geeigneter Amplituden und Phasenwahl zwischen den einzelnen Spulen 104, 404. Ferner können statische oder quasistatische Zusatzmagnetfelder 530 mit Magnetfeldgradienten innerhalb des Messvolumens 102 der Spule 104 oder der Spulen 104, 404 eingesetzt werden.

In Bezug auf das in Fig8 gezeigte Ausführungsbeispiel kann parallel und/oder in Serie zu der Spule 104 eine Anzahl von Kondensatoren, Widerständen und Spulen geschaltet werden, ebenso zu dem Widerstand 652. Hierdurch kann z. B. einfach eine manuelle Grobeinstellung realisiert werden, wenn ein oder mehrere dieser Elemente einstellbar sind.

Der Widerstand 652 kann durch eine Spule realisiert werden. Dies kann vorteilhaft sein, wenn eine geringe Phasenverschiebung der Spannungen U1-, U2~ 106, 606 gewünscht ist. Diese Spule kann so verschaltet werden, das ähnlich wie in einem Gradiometer von außen induzierte Störungen einen Spannungshub mit entgegengesetzten Vorzeichen erzeugen und sich so netto aufheben. Ebenso kann dies dazu genutzt werden mit einer niedrigeren Spannung U2~ 606 auszukommen.

Die Spule 104 kann so ausgeformt werden, dass sie in ihrem Inneren ein homogenes Feld 108 erzeugen kann. Dies ist vorteilhaft für das Vermessen von Proben 102 in z. B. Reagenzgläsern.

Die Spule 104 kann so ausgeformt sein, dass sie ein homogenes Feld 108 in einem bestimmten Bereich vor einer planen Fläche erzeugt, ebenso aber auch ein entsprechendes nicht homogenes Feld 108. Hierdurch können ausgedehnte Objekte 102 lokal durch einen kleinen Sensor 100 untersucht werden.

Eine Anzahl von solchen Messspulen 104 kann durch geeignete Anordnung, z. B. als Array lokale Messungen benachbarter Messvolumina 102, 502 gleichzeitig durchführen. Idealerweise sind dabei die einzelnen Spulen 104, 404 geometrisch, induktiv oder kapazitiv entkoppelt.

Bei einer Anordnung von Spulen 104, 404 kann eine Ortsauflösung des aufgenommenen Signals auch durch eine geeignete Wahl von Amplituden, Phasen und Frequenzen der Einzelelemente und einer geeigneten Rekonstruktion des Messsignals gewonnen werden.

Diese Anlage 100 kann auch mit extern erzeugten Magnetfeldern 530 wie statische homogene Felder oder zeitlich ändernde homogene Felder zusammenarbeiten.

Ebenso kann diese Anlage 100 mit räumlich und/oder zeitlich ändernden inhomogenen Feldern 530 zusammenarbeiten. Durch eine geeignete Rekonstruktion kann die gemessene Information räumlich zugeordnet werden.

Diese Anlage 100 dient gemäß einem Ausführungsbeispiel zum Nachweis von magnetisierbaren Partikeln, eingeschlossen solche mit linearer und mit nichtlinearer Magnetisierbarkeit bei angelegtem Magnetfeld.

Gemäß einem Ausführungsbeispiel hat die beispielsweise in Fig. 8 gezeigte Anlage 100 zwei wesentliche Betriebsmodi, einmal wird die Spannung U2~ 606 so gewählt, dass die leere Messspule 104 kein Ausgangssignal liefert, das Messsignal 110 ist hierbei dann die Spannung am Ausgang 643 bei Probe 102 im Messfeld 108. Der andere Betriebsmodus ist, dass immer die Spannung U2~ 606 so gewählt wird, dass keine Spannung am Ausgang 643 erzeugt wird, das Messsignal 110 ist dann die Differenz von der Spannung U2~ 606 bei Probe 102 im Messfeld 108 und der Spannung U2~ 606 bei keiner Probe 102 im Messfeld 108. Es ist ebenso denkbar, eine Kombination aus beiden Verfahren zu wählen. Damit kann die maximale Messspannung z. B. begrenz werden. Der resultierende Dynamikbereich ist hier die Summe der Dynamikbereiche von der Spannung U2~ 606 und der Spannungsmessung.

Eine besondere Erweiterung ist die Möglichkeit in diese Messanordnung 100 zur Leitung des Feldes 108 an einen anderen Ort magnetisch leitende Kerne 320 zu verwenden. So ist es zum Beispiel möglich, eine Nadel aus einem ferromagnetischen Material in die Spule 104 einzusetzen. Die Spule 104 erzeugt nun in dem Kern 320 einen moderaten magnetischen Fluss, an der Nadelspitze aber einen hohen. Magnetisierbare Partikel, die sich im Bereich der Nadelspitze befinden, erzeugen nun ein besonders hohes Antwortsignal, da hier die Magnetisierung am größten ist. Damit ist ein Mikroskop realisierbar.

Die beschriebenen Ausführungsbeispiele sind nur beispielhaft gewählt und können miteinander kombiniert werden.

**Patentansprüche**

1. Verfahren zur Analyse eines magnetische Partikel umfassenden Probenvolumens (102), wobei das Verfahren die folgenden Schritte umfasst:

Anlegen (701) eines elektrischen Anregungssignals (106) an eine Messspule (104), um unter Verwendung der Messspule (104) ein auf das Probenvolumen (102) einwirkendes magnetisches Feld (108) zu erzeugen, wobei ein erster Kontakt der Messspule (104) über ein erstes Element (651) mit einem ersten Anschluss (641) zum Anlegen des elektrischen Anregungssignals (106), ein zweiter Kontakt der Messspule (104) über ein zweites Element (652) mit einem zweiten Anschluss (642) zum Anlegen eines elektrischen Abgleichsignals (606) und der zweite Kontakt der Messspule (104) mit einer Messschnittstelle (643) verbunden sind, und das Anregungssignal (106) an den ersten Anschluss (641) und das Abgleichsignal (606) an den zweiten Anschluss (642) angelegt wird;

Erfassen (703) eines von der Induktivität der Messspule (104) abhängigen elektrischen Messsignals (110), wobei das Abgleichsignal (606) als das Messsignal (110) erfasst wird und das Abgleichsignal (606) ausgeformt ist, um unabhängig davon, ob das von der Messspule (104) erzeugte magnetische Feld (108) auf das Probenvolumen (102) oder ein Referenzvolumen einwirkt, an der Messschnittstelle (643) eine vorbestimmte Referenzspannung einzustellen; und

Analysieren (705) einer magnetischen Permeabilität des Probenvolumens (102) unter Verwendung des Messsignals (110).

2. Verfahren gemäß Anspruch 1, mit einem Schritt des Vergleichens des Messsignals (110) mit einem Referenzsignal, um die magnetische Permeabilität des Probenvolumens (102) zu bestimmen, wobei das Referenzsignal ein weiteres elektrisches Messsignal (110) repräsentiert, das von der Induktivität der Messspule (104) abhängig ist, während die Messspule (104) ein auf ein Referenzvolumen einwirkendes magnetisches Feld (108) erzeugt.

3. Verfahren gemäß einem der vorangegangenen Ansprüche, mit einem Schritt des Anlegens eines weiteren elektrischen Anregungssignals (406) an eine weitere Messspule (404), um unter Verwendung der weiteren Messspule (404) ein auf ein weiteres Probenvolumen (502) einwirkendes weiteres magnetisches Feld (408) zu erzeugen, und mit einem Schritt des Erfassens (703) eines von der Induktivität der weiteren Messspule (404) abhängigen weiteren elektrischen Messsignals (410), wobei das weitere Messsignal (410) aufgrund der Abhängigkeit der Induktivität der weiteren Messspule (404) von der magnetischen Permeabilität des weiteren Probenvolumens (502) eine Analyse der magnetischen Permeabilität des weiteren Probenvolumens (502) ermöglicht.

4. Verfahren gemäß Anspruch 3, mit einem Schritt des Bestimmens einer Verteilung der magnetischen Partikel in dem Probenvolumen (102) und dem weiteren Probenvolumen (502) unter Verwendung des Messsignals (110), des weiteren Messsignals (410) sowie einer Information über eine Position des Probenvolumens (102), einer Position des weiteren Probenvolumens (502), einer Information über eine Charakteristik des Anregungssignals (106) und einer Information über eine Charakteristik des weiteren Anregungssignals (406).

5. Verfahren gemäß einem der vorangegangenen Ansprüche, mit einem Schritt des Beaufschlagens des Probenvolumens (102) mit einem inhomogenen zusätzlichen Magnetfeld (530) und wobei im Schritt des Analysierens (705) eine Verteilung der magnetischen Partikel in dem Probenvolumen (102) unter Verwendung des Messsignals (110) sowie einer Information über einen örtlichen Verlauf des zusätzlichen Magnetfelds (530) innerhalb des Probenvolumens (102) bestimmt wird.

6. Verfahren gemäß Anspruch 5, bei dem die Schritte des Anlegens (701) und des Erfassens (703) sowie des Beaufschlagens mehrmals ausgeführt werden, wobei der örtliche Verlauf des zusätzlichen Magnetfelds in den mehrmaligen Schritten des Beaufschlagens des Probenvolumens (102) verändert wird und wobei im Schritt des Analysierens (705) die Verteilung der magnetischen Partikel unter Verwendung der in den mehrmaligen Schritten des Erfassens (703) erfassten Messsignale (110) und einer Information über eine Veränderung des örtlichen Verlaufs des zusätzlichen Magnetfelds (530) während der mehrmaligen Schritte des Beaufschlagens bestimmt wird.

7. Vorrichtung (100) zur Analyse eines magnetische Partikel umfassenden Probenvolumens (102), wobei die Vorrichtung die folgenden Merkmale aufweist:

eine Messspule (104) die ausgebildet ist, um ansprechend auf ein Anlegen eines elektrischen Anregungssignals (106) an die Messspule (104) ein auf das Probenvolumen (102) einwirkendes magnetisches Feld (108) zu erzeugen, wobei ein erster Kontakt der Messspule (104) über ein erstes Element (651) mit einem ersten Anschluss (641) zum Anlegen des elektrischen Anregungssignals (106), ein zweiter Kontakt der Messspule (104) über ein zweites Element (652) mit einem zweiten Anschluss (642) zum Anlegen eines elektrischen Abgleichsignals (606) und der zweite Kontakt der Messspule (104) mit einer Messschnittstelle (643) verbunden sind;
eine Anregungseinrichtung (114), die ausgebildet ist, das Anregungssignal (106) an den ersten Anschluss (641) und das Abgleichsignal (606) an den zweiten Anschluss (642) anzulegen, wobei das Abgleichsignal (606) ausgeformt ist, um unabhängig davon, ob das von der Messspule (104) erzeugte magnetische Feld (108) auf das Probenvolumen (102) oder ein Referenzvolumen einwirkt, an der Messschnittstelle (643) eine vorbestimmte Referenzspannung einzustellen; und

eine Auswerteeinrichtung (112), die ausgebildet ist, eine magnetischen Permeabilität des Probenvolumens (102) unter Verwendung des als Messsignals (110) auswertbaren Ableichsignals (606) zu analysieren.

8. Vorrichtung (100) gemäß Anspruch 7, bei der das Probenvolumen (102) im Inneren der Messspule (104) angeordnet ist.

9. Vorrichtung (100) gemäß Anspruch 7, mit einem Spulenkern (320), der durch die Messspule (104) geführt ist, wobei ein Ende des Spulenkerns (320) als eine Spitze zum Anlegen an das Probenvolumen (102) ausgeführt ist.

10. Vorrichtung (100) gemäß einem der Ansprüche 7 bis 9, mit einer Auswerteeinrichtung (112), die ausgebildet ist, um die magnetische Permeabilität des Probenvolumens (102) unter Verwendung des Messsignals (110) zu bestimmen.

11. Vorrichtung gemäß Anspruch 7, bei der das erste Element (651) ein Kondensator oder eine elektrische Durchgangsleitung und das zweite Element (642) ein Widerstand, eine Spule oder eine elektrische Durchgangsleitung ist.

12. Vorrichtung gemäß einem der Ansprüche 7 bis 11, mit einer weiteren Messspule (404), die ausgebildet ist, um ansprechend auf ein Anlegen eines weiteren elektrischen Anregungssignals (406) an die weitere Messspule (404) ein auf ein weiteres Probenvolumen (502) einwirkendes weiteres magnetisches Feld (408) zu erzeugen, mit einer weiteren Messschnittstelle zum Erfassen eines von der Induktivität der weiteren Messspule (404) abhängigen weiteren elektrischen Messsignals (410), wobei das weitere Messsignal (410) aufgrund der Abhängigkeit der Induktivität der weiteren Messspule (404) von der magnetischen Permeabilität des weiteren Probenvolumens (502) eine Analyse der magnetischen Permeabilität des weiteren Probenvolumens (502) ermöglicht.

13. Vorrichtung gemäß Anspruch 12, bei dem das Probenvolumen (102) und das weitere Probenvolumen (502) zumindest teilweise überlappen.

**Claims**

1. A method of analyzing a sample volume (102) comprising magnetic particles, the method comprising the steps of:

applying (701) an electrical excitation signal (106) to a measurement coil (104) to generate a magnetic field (108) acting on the sample volume (102) using the measurement coil (104), wherein a first contact of the measurement coil (104) via a first element (651) with a first terminal (641) for applying the electrical excitation signal (106), a second contact of the measurement coil (104) is connected via a second element (652) to a second terminal (642) for applying an electrical adjustment signal (606), and the second contact of the measurement coil (104) is connected to a measurement interface (643), and the excitation signal (106) is applied to the first terminal (641) and the adjustment signal (606) is applied to the second terminal (642); detecting (703) an electrical measurement signal (110) dependent on the inductance of the measurement coil (104), wherein the adjustment signal (606) is detected as the measurement signal (110) and the adjustment signal (606) is shaped to set a predetermined reference voltage at the measurement interface (643) regardless of whether the magnetic field (108) generated by the measurement coil (104) acts on the sample volume (102) or a reference volume; and analyzing (705) a magnetic permeability of the sample volume (102) using the measurement signal (110).

2. Method according to claim 1, comprising a step of comparing the measurement signal (110) with a reference signal to determine the magnetic permeability of the sample volume (102), wherein the reference signal represents a further electrical measurement signal (110) that is dependent on the inductance of the measurement coil (104) while the measurement coil (104) generates a magnetic field (108) acting on a reference volume.

3. A method according to any one of the preceding claims, comprising a step of applying a further electrical excitation signal (406) to a further measurement coil (404) to generate a further magnetic field (408) acting on a further sample volume (502) using the further measurement coil (404), and with a step of detecting (703) a further electrical measurement signal (410) dependent on the inductance of the further measurement coil (404), wherein the further measurement signal (410) enables an analysis of the magnetic permeability of the further sample volume (502) due to the dependence of the inductance of the further measurement coil (404) on the magnetic permeability of the further sample volume (502).

4. Method according to claim 3, comprising a step of determining a distribution of the magnetic particles in the sample volume (102) and the further sample volume (502) using the measurement signal (110), the further measurement signal (410) and information about a position of the sample volume (102), a position of the further sample volume (502), information about a characteristic of the excitation signal (106) and information about a characteristic of the further excitation signal (406).

5. Method according to any one of the preceding claims, comprising a step of exposing the sample volume (102) to an inhomogeneous additional magnetic field (530) and wherein in the step of analyzing (705) a distribution of the magnetic particles in the sample volume (102) is determined using the measurement signal (110) and information about a local course of the additional magnetic field (530) within the sample volume (102).

6. Method according to claim 5, wherein the steps of applying (701) and detecting (703) and exposing are performed a plurality of times, wherein the local variation of the additional magnetic field is changed in the multiple steps of exposing the sample volume (102), and wherein in the step of analyzing (705) the distribution of the magnetic particles is determined using the measurement signals (110) detected in the multiple steps of detecting (703) and information about a change in the local variation of the additional magnetic field (530) during the multiple steps of exposing.

7. Apparatus (100) for analyzing a sample volume (102) comprising magnetic particles, the device having the following features:

a measurement coil (104) configured to generate a magnetic field (108) acting on the sample volume (102) in response to application of an electrical excitation signal (106) to the measurement coil (104), wherein a first contact of the measurement coil (104) is made via a first element (651) having a first terminal (641) for application of the electrical excitation signal (106), a second contact of the measurement coil (104) being connected via a second element (652) to a second terminal (642) for applying an electrical adjustment signal (606), and the second contact of the measurement coil (104) being connected to a measurement interface (643);
an excitation device (114) configured to apply the excitation signal (106) to the first terminal (641) and the adjustment signal (606) to the second terminal (642), wherein the adjustment signal (606) is shaped to set a predetermined reference voltage at the measurement interface (643) regardless of whether the magnetic field (108) generated by the measurement coil (104) acts on the sample volume (102) or a reference volume; and
an evaluation device (112) which is designed to analyze a magnetic permeability of the sample volume (102) using the calibration signal (606) which can be evaluated as a measurement signal (110).

8. Apparatus (100) according to claim 7, wherein the sample volume (102) is arranged inside the measuring coil (104).

9. Apparatus (100) according to claim 7, comprising a coil core (320) guided by the measurement coil (104), wherein one end of the coil core (320) is configured as a tip for application to the sample volume (102).

10. Apparatus (100) according to any one of claims 7 to 9, comprising an evaluation device (112) configured to determine the magnetic permeability of the sample volume (102) using the measurement signal (110).

11. Apparatus according to claim 7, wherein the first element (651) is a capacitor or an electrical continuity line and the second element (642) is a resistor, a coil or an electrical continuity line.

12. Apparatus according to any one of claims 7 to 11, comprising a further measurement coil (404) arranged to generate a further magnetic field (408) acting on a further sample volume (502) in response to application of a further electrical excitation signal (406) to the further measurement coil (404), having a further measuring interface for detecting a further electrical measuring signal (410) which is dependent on the inductance of the further measuring coil (404), the further measuring signal (410) making it possible to analyze the magnetic permeability of the further sample volume (502) on the basis of the dependence of the inductance of the further measuring coil (404) on the magnetic permeability of the further sample volume (502).

13. Apparatus according to claim 12, wherein the sample volume (102) and the further sample volume (502) at least partially overlap.

**Revendications**

1. Procédé d'analyse d'un volume d'échantillon (102) comprenant des particules magnétiques, la méthode comprenant les étapes suivantes:

   appliquer (701) un signal d'excitation électrique (106) à une bobine de mesure (104) pour générer un champ magnétique (108) agissant sur le volume d'échantillon (102) à l'aide de la bobine de mesure (104), dans laquelle un premier contact de la bobine de mesure (104) via un premier élément (651) avec une première borne (641) pour appliquer le signal d'excitation électrique (106), un second contact de la bobine de mesure (104) est connecté via un second élément (652) à une seconde borne (642) pour appliquer un signal d'ajustement électrique (606), et le second contact de la bobine de mesure (104) est connecté à une interface de mesure (643), et le signal d'excitation (106) est appliqué à la première borne (641) et le signal d'ajustement (606) est appliqué à la seconde borne (642);
   détecter (703) un signal de mesure électrique (110) dépendant de l'inductance de la bobine de mesure (104), le signal d'ajustement (606) étant détecté en tant que signal de mesure (110) et le signal d'ajustement (606) étant façonné pour définir une tension de référence prédéterminée au niveau de l'interface de mesure (643), que le champ magnétique (108) généré par la bobine de mesure (104) agisse sur le volume de l'échantillon (102) ou sur un volume de référence; et
   analyser (705) une perméabilité magnétique du volume de l'échantillon (102) à l'aide du signal de mesure (110).

2. Procédé selon la revendication 1, comprenant une étape de comparaison du signal de mesure (110) avec un signal de référence pour déterminer la perméabilité magnétique du volume de l'échantillon (102), le signal de référence représentant un autre signal de mesure électrique (110) qui dépend de l'inductance de la bobine de mesure (104) tandis que la bobine de mesure (104) génère un champ magnétique (108) agissant sur un volume de référence.

3. Procédé selon l'une quelconque des revendications précédentes, comprenant une étape consistant à appliquer un autre signal d'excitation électrique (406) à une autre bobine de mesure (404) pour générer un autre champ magnétique (408) agissant sur un autre volume d'échantillon (502) à l'aide de l'autre bobine de mesure (404), et avec une étape de détection (703) d'un autre signal de mesure électrique (410) dépendant de l'inductance de la bobine de mesure supplémentaire (404), dans laquelle le signal de mesure supplémentaire (410) permet une analyse de la perméabilité magnétique du volume d'échantillon supplémentaire (502) en raison de la dépendance de l'inductance de la bobine de mesure supplémentaire (404) par rapport à la perméabilité magnétique du volume d'échantillon supplémentaire (502).

4. Procédé selon la revendication 3, comprenant une étape de détermination d'une distribution des particules magnétiques dans le volume d'échantillon (102) et le volume d'échantillon supplémentaire (502) en utilisant le signal de mesure (110), le signal de mesure supplémentaire (410) et des informations sur une position du volume d'échantillon (102), une position du volume d'échantillon supplémentaire (502), des informations sur une caractéristique du signal d'excitation (106) et des informations sur une caractéristique du signal d'excitation supplémentaire (406).

5. Procédé selon l'une quelconque des revendications précédentes, comprenant une étape d'exposition du volume d'échantillon (102) à un champ magnétique supplémentaire inhomogène (530) et dans lequel, lors de l'étape d'analyse (705), une distribution des particules magnétiques dans le volume d'échantillon (102) est déterminée à l'aide du signal de mesure (110) et d'informations sur un parcours local du champ magnétique supplémentaire (530) à l'intérieur du volume d'échantillon (102).

6. Procédé selon la revendication 5, dans lequel les étapes d'application (701), de détection (703) et d'exposition sont effectuées plusieurs fois, dans lequel la variation locale du champ magnétique supplémentaire est modifiée au cours des multiples étapes d'exposition du volume d'échantillon (102), et dans lequel, à l'étape d'analyse (705), la distribution des particules magnétiques est déterminée à l'aide des signaux de mesure (110) détectés au cours des multiples étapes de détection (703) et des informations relatives à une modification de la variation locale du champ magnétique supplémentaire (530) au cours des multiples étapes d'exposition.

7. Appareil (100) pour l'analyse d'un volume d'échantillon (102) comprenant des particules magnétiques, le dispositif ayant les caractéristiques suivantes:

une bobine de mesure (104) configurée pour générer un champ magnétique (108) agissant sur le volume d'échantillon (102) en réponse à l'application d'un signal d'excitation électrique (106) à la bobine de mesure (104), dans laquelle un premier contact de la bobine de mesure (104) est réalisé par l'intermédiaire d'un premier élément (651) ayant une première borne (641) pour l'application du signal d'excitation électrique (106), un second contact de la bobine de mesure (104) est connecté via un second élément (652) à une seconde borne (642) pour l'application d'un signal d'ajustement électrique (606), et le second contact de la bobine de mesure (104) est connecté à une interface de mesure (643) ;

un dispositif d'excitation (114) configuré pour appliquer le signal d'excitation (106) à la première borne (641) et le signal de réglage (606) à la seconde borne (642), le signal de réglage (606) étant configuré pour établir une tension de référence prédéterminée au niveau de l'interface de mesure (643), que le champ magnétique (108) généré par la bobine de mesure (104) agisse sur le volume de l'échantillon (102) ou sur un volume de référence ; et

un dispositif d'évaluation (112) conçu pour analyser la perméabilité magnétique du volume de l'échantillon (102) à l'aide du signal d'étalonnage (606) qui peut être évalué en tant que signal de mesure (110).

8. Appareil (100) selon la revendication 7, dans lequel le volume d'échantillon (102) est disposé à l'intérieur de la bobine de mesure (104).

9. Appareil (100) selon la revendication 7, comprenant un noyau de bobine (320) guidé par la bobine de mesure (104), dans lequel une extrémité du noyau de bobine (320) est configurée comme une pointe pour l'application au volume d'échantillon (102).

10. Appareil (100) selon l'une quelconque des revendications 7 à 9, comprenant un dispositif d'évaluation (112) configuré pour déterminer la perméabilité magnétique du volume d'échantillon (102) à l'aide du signal de mesure (110).

11. Appareil selon la revendication 7, dans lequel le premier élément (651) est un condensateur ou une ligne de continuité électrique et le second élément (642) est une résistance, une bobine ou une ligne de continuité électrique.

12. Appareil selon l'une des revendications 7 à 11, comprenant une autre bobine de mesure (404) conçue pour générer un autre champ magnétique (408) agissant sur un autre volume d'échantillon (502) en réponse à l'application d'un autre signal d'excitation électrique (406) à l'autre bobine de mesure (404), ayant une autre interface de mesure pour détecter un autre signal de mesure électrique (410) qui dépend de l'inductance de la bobine de mesure supplémentaire (404), le signal de mesure supplémentaire (410) permettant d'analyser la perméabilité magnétique du volume d'échantillon supplémentaire (502) sur la base de la dépendance de l'inductance de la bobine de mesure supplémentaire (404) par rapport à la perméabilité magnétique du volume d'échantillon supplémentaire (502).

13. Appareil selon la revendication 12, dans lequel le volume d'échantillon (102) et le volume d'échantillon supplémentaire (502) se chevauchent au moins partiellement.

FIG 1

FIG 2

100

104

114

106

110

108

320

102

FIG 3

100

112

104

114

110

106

108

408

102

410

406

404

FIG 4

FIG 5

FIG 6

FIG 7

FIG 8

FIG 9

FIG 10

# IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120164916 A1
- WO 2008145813 A1
- WO 2007122293 A1

**In der Beschreibung aufgeführte Nicht-Patentliteratur**

- **GLEICH ; WEIZENECKER.** *Nature,* 2005, vol. 435 (30), 1214